(19) Europäisches Patentamt
European Patent Office
Office européen des brevets

(11) Veröffentlichungsnummer : **0 359 953 B1**

(12) # EUROPÄISCHE PATENTSCHRIFT

(45) Veröffentlichungstag der Patentschrift : **22.01.92 Patentblatt 92/04**

(51) Int. Cl.$^5$ : **C07C 39/17,** C08G 64/00

(21) Anmeldenummer : **89114067.5**

(22) Anmeldetag : **29.07.89**

(54) **Dihydroxydiphenylcycloalkane, ihre Herstellung und ihre Verwendung zur Herstellung von hochmolekularen Polycarbonaten.**

Die Akte enthält technische Angaben, die nach dem Eingang der Anmeldung eingereicht wurden und die nicht in dieser Patentschrift enthalten sind.

(30) Priorität : **12.08.88 DE 3827434**
**12.08.88 DE 3827435**
**23.09.88 DE 3832396**
**01.11.88 DE 3837090**
**23.03.89 DE 3909601**

(43) Veröffentlichungstag der Anmeldung : **28.03.90 Patentblatt 90/13**

(45) Bekanntmachung des Hinweises auf die Patenterteilung : **22.01.92 Patentblatt 92/04**

(84) Benannte Vertragsstaaten : **AT BE CH DE ES FR GB GR IT LI LU NL SE**

(56) Entgegenhaltungen :
**EP-A- 0 023 571**
**EP-A- 0 089 801**
**EP-A- 0 166 834**
**EP-A- 0 249 963**
**EP-A- 0 274 092**
**US-A- 2 069 560**
**US-A- 2 069 573**
**US-A- 2 342 294**

(56) Entgegenhaltungen :
**US-A- 2 538 725**
**US-A- 2 883 365**
**US-A- 4 180 651**
**US-A- 4 637 971**

(73) Patentinhaber : **BAYER AG**
**W-5090 Leverkusen 1 Bayerwerk (DE)**

(72) Erfinder : **Freitag, Dieter, Dr.**
**Hasenheide 10**
**W-4150 Krefeld (DE)**
Erfinder : **Westeppe, Uwe, Dr.**
**Vogelskamp 72**
**W-4020 Mettmann 2 (DE)**
Erfinder : **Wulff, Claus, H., Dr.**
**Richard-Strauss-Strasse 21**
**W-4150 Krefeld (DE)**
Erfinder : **Fritsch, Karl-Herbert, Dr.**
**Hackberg 25**
**W-5060 Bergisch-Gladbach 1 (DE)**
Erfinder : **Casser, Carl, Dr.**
**Herseler Strasse 14**
**W-5300 Bonn (DE)**
Erfinder : **Weymans, Günther, Dr.**
**Karl-Arnold-Strasse 4**
**W-5090 Leverkusen (DE)**
Erfinder : **Schrader, Lutz, Dr.**
**Krakauer Strasse 86**
**W-4150 Krefeld (DE)**
Erfinder : **Waldenrath, Werner, Dr.**
**Maastrichter Strasse 40**
**W-5000 Köln 1 (DE)**

EP 0 359 953 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der vorliegenden Erfindung sind Dihydroxydiphenylcycloalkane der Formel (I)

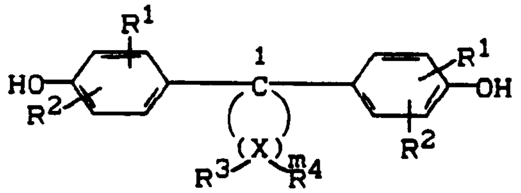

(I),

worin

| | |
|---|---|
| $R^1$ und $R^2$ | unabhängig voneinander Wasserstoff, Halogen, bevorzugt Chlor oder Brom, $C_1$-$C_8$-Alkyl, $C_5$-$C_6$-Cycloalkyl, $C_6$-$C_{10}$-Aryl, bevorzugt Phenyl, und $C_7$-$C_{12}$-Aralkyl, bevorzugt Phenyl-$C_1$-$C_4$-Alkyl, insbesondere Benzyl, |
| m | 4 oder 5, |
| $R^3$ und $R^4$, | für jedes X individuell wählbar, unabhängig voneinander Wasserstoff oder $C_1$-$C_6$-Alkyl und |
| X | Kohlenstoff bedeuten, |

mit der Maßgabe, daß an mindestens einem Atom X $R^3$ und $R^4$ gleichzeitig Alkyl bedeuten.

Bevorzugt sind an 1-2 Atomen X, insbesondere nur an einem Atom X, $R^3$ und $R^4$ gleichzeitig Alkyl. Bevorzugter Alkylrest ist Methyl ; die X-Atome in α-Stellung zu dem di-phenyl-substituierten C-Atom (C-1) sind bevorzugt nicht dialkylsubstituiert, dagegen ist die Alkyl-disubstitution in β-Stellung zu C-1 bevorzugt. Besonders bevorzugt ist, daß ein X-Atom in β-Stellung dialkylsubstituiert, und ein X Atom in β'-Stellung monoalkylsubstituiert ist.

Insbesondere sind Gegenstand der Erfindung Dihydroxydiphenylcycloalkane mit 5 und 6 Ring-C-Atomen im cycloaliphatischen Rest (m = 4 oder 5 in Formel (I) wie beispielsweise die Diphenole der Formeln

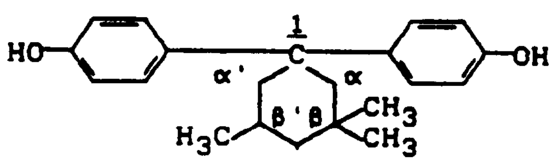

(II)

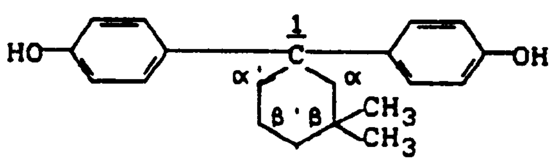

(III) und

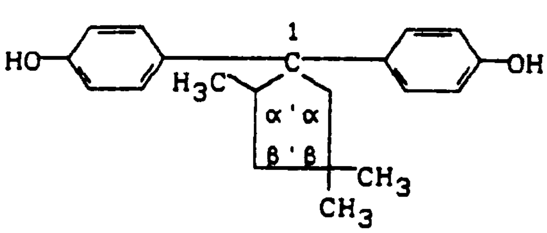

(IV),

wobei das 1,1-Bis-(4-hydroxyphenyl)-3.3.5-trimethylcyclohexan (Formel II) besonders bevorzugt ist.

Die erfindungsgemäßen Dihydroxydiphenylcycloalkane der Formel (I) können in an sich bekannter Weise durch Kondensation von Phenolen der Formel (V)

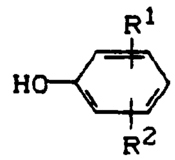

(V)

und Ketonen der Formel (VI)

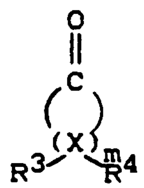

(VI)

hergestellt werden, wobei in den Formeln (V) und (VI) X, $R^1$, $R^2$, $R^3$, $R^4$ und m die für Formel (I) angegebene Bedeutung haben.

Die Phenole der Formel (V) sind entweder literaturbekannt oder nach literaturbekannten Verfahren erhältlich (siehe beispielsweise für Kresole und Xylenole, Ullmanns Encyklopädie der technischen Chemie 4. neubearbeitete und erweiterte Auflage Band 15, Seiten 61-77, Verlag Chemie-Weinheim-New York 1978 ; für Chlorphenole, Ullmanns Encyklopädie der technischen Chemie, 4. Auflage, Verlag Chemie, 1975, Band 9, Seiten 573-582 ; und für Alkylphenole, Ullmanns Encyklopädie der technischen Chemie, 4. Auflage, Verlag Chemie 1979, Band 18, Seiten 191-214).

Beispiele für geeignete Phenole der Formel (V) sind : Phenol, o-Kresol, m-Kresol, 2,6-Dimethylphenol, 2-Chlorphenol, 3-Chlorphenol, 2,6-Dichlorphenol, 2-Cyclohexylphenol, 2,6-Diphenylphenol und o-Benzylphenol.

Die Ketone der Formel (VI) sind literaturbekannt (siehe beispielsweise) Beilsteins Handbuch der Organischen Chemie, 7. Band, 4. Auflage, Springer-Verlag, Berlin, 1925 und die entsprechenden Ergänzungsbände 1 bis 4, und J. Am. Chem. Soc. Vol 79 (1957), Seiten 1488-1492, US-PS 2692289, Allen et al., J. Chem. Soc., (1959), 2186-2192 und J. Org. Chem. Vol. 38, (1973), Seiten 4431-4435, J. Am. Chem. Soc. 87, (1965), Seiten 1353-1364. Ein allgemeines Verfahren zur Herstellung von Ketonen der Formel (VI) ist beispielsweise in "Organikum, 15. Auflage, 1977, VEB-Deutscher Verlag der Wissenschaften, Berlin, beispielsweise Seite 698, beschrieben.

Beispiele für bekannte Ketone der Formel (VI) sind :
3,3-Dimethylcyclopentanon, 3,3-Dimethylcyclohexanon, 4,4-Dimethylcyclohexanon, 3-Ethyl-3-Methylcyclopentanon, 2,3,3-Trimethylcyclopentanon, 3,3,4-Trimethylcyclopentanon, 3,3-Dimethylcycloheptanon, 4,4,-Dimethylcycloheptanon, 3-Ethyl-3-methylcyclohexanon, 4-Ethyl-4-methylcyclohexanon, 2,3,3-Trimethylcyclohexanon, 2,4,4-Trimethylcyclohexanon, 3,3,4-Trimethylcyclohexanon, 3,3,5-Trimethylcyclohexanon, 3,4,4-Trimethylcyclohexanon, 2,3,3,4-Tetramethylcyclopentanon, 4-Ethyl-2,3,4-trimethylcyclopentanon, 3-Ethyl-4-isopropyl-3-methyl-cyclopentanon, 4-sec. Butyl-3,3-dimethylcyclopentanon, 2-Isopropyl-3,3,4-trimethylcyclopentanon, 3-Ethyl-4-isopropyl-3-methyl-cyclohexanon, 4-Ethyl-3-isopropyl-4-methylcyclohexanon, 3-sec.Butyl-4,4-dimethylcyclohexanon, 2-Butyl-3,3,4-trimethylcyclopentanon, 2-Butyl-3,3,4-trimethylcyclohexanon, 4-Butyl-3,3,5-trimethylcyclohexanon und 3-Isohexyl-3-methylcyclohexanon.

Beispiele für bevorzugte Ketone sind

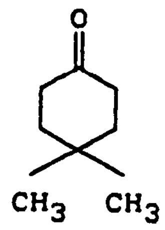

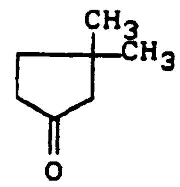

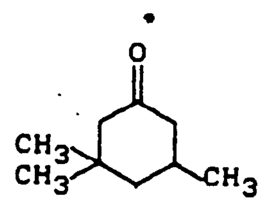

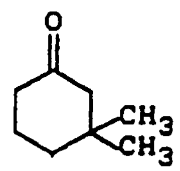

und

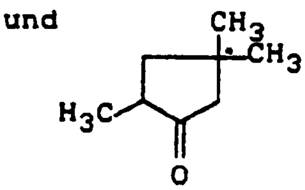

Zur Bisphenolherstellung werden im allgemeinen 2 bis 10 Mol, vorzugsweise 2,5 bis 6 Mol Phenol (V) pro Mol Keton (VI), verwendet. Bevorzugte Reaktionszeiten betragen 1 bis 100 Stunden. Im allgemeinen arbeitet man bei Temperaturen von – 30°C bis 300°C, vorzugsweise von – 15°C bis 150°C und bei Drücken von 1 bis 20 bar, vorzugsweise von 1 bis 10 bar.

Die Kondensation wird im allgemeinen in Gegenwart saurer Katalysatoren durchgeführt. Beispiele sind Chlorwasserstoff, Bromwasserstoff, Fluorwasserstoff, Bortrifluorid, Aluminiumtrichlorid, Zinkdichlorid, Titantetrachlorid, Zinntetrachlorid, Phosphorhalogenide, Phosphorpentoxid, Phosphorsäure, konzentrierte Salzsäure oder Schwefelsäure sowie Mischungen aus Essigsäure und Acetanhydrid. Die Verwendung saurer Ionenaustauscher ist ebenfalls möglich.

Weiterhin kann die Umsetzung durch Zugabe von Co-Katalysatoren wie $C_1$-$C_{18}$-Alkyl-Mercaptanen, Schwefelwasserstoff, Thiophenolen, Thiosäuren und Dialkylsulfiden bevorzugt in Mengen von 0,01-0,4 Mol/Mol Keton, insbesondere 0,05-0,2 Mol/Mol Keton beschleunigt werden.

Die Kondensation kann ohne Lösungsmittel oder in Gegenwart eines inerten Lösungsmittels (z.B. aliphatischer und aromatischer Kohlenwasserstoff, Chlorkohlenwasserstoff) durchgeführt werden.

In den Fällen, in denen der Katalysator gleichzeitig als wasserentziehendes Mittel fungiert, ist es nicht erforderlich, zusätzlich wasserentziehende Mittel einzusetzen, letzteres ist jedoch zur Erzielung guter Umsätze in jedem Fall dann vorteilhaft, wenn der eingesetzte Katalysator das Reaktionswasser nicht bindet.

Geeignete wasserentziehende Mittel sind beispielsweise Acetanhydrid, Zeolithe, Polyphosphorsäure und Phosphorpentoxid.

Gegenstand der vorliegenden Erfindung ist somit außerdem ein Verfahren zur Herstellung der Dihydroxydiphenylcycloalkane der Formel (I)

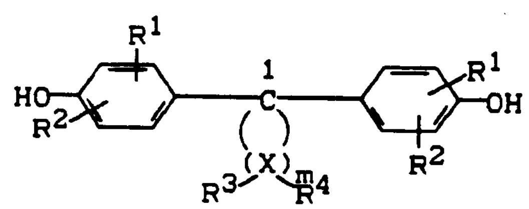

(I),

worin

| | |
|---|---|
| $R^1$ und $R^2$ | unabhängig voneinander Wasserstoff, Halogen, bevorzugt Chlor oder Brom, $C_1$-$C_8$-Alkyl, $C_5$-$C_6$-Cycloalkyl, $C_6$-$C_{10}$-Aryl, bevorzugt Phenyl, und $C_7$-$C_{12}$-Aralkyl, bevorzugt Phenyl-$C_1$-$C_4$-Alkyl, insbesondere Benzyl, |
| m | 4 oder 5 sind, |
| $R^3$ und $R^4$, | für jedes X individuell wählbar, unabhängig voneinander Wasserstoff oder $C_1$-$C_6$-Alkyl und |
| X | Kohlenstoff ist |

mit der Maßgabe, daß an mindestens einem Atom X $R^3$ und $R^4$ gleichzeitig Alkyl bedeuten, das dadurch gekennzeichnet ist, daß man Phenole der Formel (V)

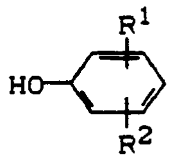

(V)

worin
$R^1$ und $R^2$ die für Formel (I) angegebene Bedeutung haben,
mit Ketonen der Formel (VI)

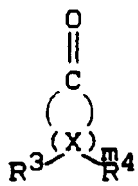

(VI)

worin
X, m, $R^3$ und $R^4$ die für Formel (I) angegebene Bedeutung haben,
im Molverhältnis (V) : (VI) von 2 : 1 bis 10 : 1, vorzugsweise von 2,5 : 1 bis 6 : 1 bei – 30°C bis 300°C, vorzugsweise – 15°C bis 150°C und bei Drücken von 1 bis 20 bar, vorzugsweise von 1 bis 10 bar in Anwesenheit saurer Katalysatoren und gegebenenfalls in Anwesenheit von Co-Katalysatoen und/oder Lösungsmitteln und/oder wasserentziehenden Mitteln umsetzt.

Bevorzugt sind in Formel (I) an 1-2 Atomen X, insbesondere nur an einem Atom X, $R^3$ und $R^4$ gleichzeitig Alkyl. Bevorzugter Alkylrest ist Methyl ; ebenfalls verwendbar sind Ethyl oder $C_3$-$C_6$-Alkylreste, die geradkettig oder verzweigt sein können. Die X-Atome in α-Stellung zu dem di-phenylsubstituierten C-Atom (C-1) sind bevorzugt nicht dialkylsubstituiert, dagegen ist die Disubstitution mit Alkyl in β-Stellung in C-1 bevorzugt. Besonders bevorzugt ist, daß ein x-Atom in β-Stellung dialkylsubstituiert und ein X-Atom in β′-Stellung monoalkylsubstituiert ist.

In manchen Fällen verläuft die Reaktion nicht ganz einheitlich, d.h. es können mehrere, verschiedene Produkte entstehen, so daß die gewünschte Verbindung zunächst aus einem Gemisch isoliert werden muß. Für Einzelheiten der Kondensation sei auf Schnell, Chemistry und Physics of Polycarbonates, Interscience Publishers, New York 1964 verwiesen. Manchmal kann die Reaktion durch Wahl entsprechender Katalysatoren und Reaktionsbedingungen so gesteuert werden, daß die gewünschte Verbindung ausfällt oder auskristallisiert, was deren Isolierung erleichtert.

Beispiel A.1

Herstellung des Diphenols der Formel (II)

In einem 1 l-Rundkolben mit Rührer, Tropftrichter, Thermometer, Rückflußkühler und Gaseinleitungsrohr werden 7,5 Mol (705 g) Phenol und 0,15 Mol (30,3 g) Dodecylthiol vorgelegt und bei 28 bis 30°C mit trockenem HCl-Gas gesättigt. Zu dieser Lösung werden innerhalb von 3 Stunden eine Lösung von 1,5 Mol (210 g) Dihydroisophoron (3,3,5-Trimethyl-cyclohexan-1-on) und 1,5 Mol (151 g) Phenol zugetropft, wobei weiter HCl-Gas in die Reaktionslösung geleitet wird. Nach Ende des Zutropfens leitet man für weitere 5 Stunden HCl-Gas ein. Man läßt 8 Stunden bei Zimmertemperatur nachreagieren. Anschließend wird das überschüssige Phenol durch Wasserdampfdestillation entfernt. Der verbleibende Rückstand wird zweimal mit Petrolether (60-90) und einmal mit Methylenchlorid heiß extrahiert und abfiltriert.

Ausbeute : 370 g
Schmelzpunkt : 205 bis 207°C

Beispiel A.2

Herstellung des Diphenols der Formel (II)

In einer Rührapparatur mit Rührer, Thermometer, Rückflußkühler und Gaseinleitungsrohr werden 1692 g (18 Mol) Phenol, 60,6 g (0,3 Mol) Dodecylthiol und 420 g (3 Mol) Dihydroisophoron (3,3,5-Trimethyl-cyclohexan-1-on) bei 28-30°C vorgelegt. In diese Lösung wird bei 28-30°C 5 h trockenes HCl-Gas eingeleitet. Man läßt ca. 10 h bei 28-30°C nachreagieren. Nach 95%igem Umsatz des Ketons (GC-Kontrolle) gibt man zum Reaktionsgemisch 2,5 l Wasser und stellt durch Zugabe von 45%iger NaOH-Lösung einen pH-Wert von 6 ein. Das Reaktionsgemisch wird eine Stunde bei 80°C gerührt und anschließend auf 25°C abgekühlt. Die wäßrige Phase wird abdekantiert und der verbleibende Rückstand mit Wasser bei 80°C gewaschen. Das erhaltene Rohprodukt wird abfiltriert und jeweils zweimal mit n-Hexan und Methylenchlorid heiß extrahiert und filtriert. Der Rückstand wird zweimal aus Xylol umkristallisiert.

Ausbeute : 753 g
Schmelzpunkt : 209-211°C.

Beispiel A.3

Herstellung des Diphenols der Formel (II)

In einer Rührapparatur mit Rührer, Thermometer, Rückflußkühler und Gaseinleitungsrohr werden 564 g (6 Mol) Phenol, 10,8 g (0,12 Mol) Butanthiol und 140 g (1 Mol) Dihydroisophoron (3,3,5-Trimethyl-cyclohexan-1-on) bei 30°C vorgelegt. Bei dieser Temperatur werden 44 g 37%ige HCl zugegeben. Das Reaktionsgemisch wird bei 28-30°C ca. 70 h gerührt. Nach 95%igem Umsatz des Ketons (GC-Kontrolle) gibt man zum Reaktionsgemisch 2 l Wasser und stellt durch Zugabe von 45%iger NaOH-Lösung einen pH-Wert von 6 ein. Das Reaktionsgemisch wird eine Stunde bei 80°C gerührt und anschließend auf 25°C abgekühlt. Die wäßrige Phase wird abdekantiert und der verbleibende Rückstand mit Wasser bei 80°C gewaschen. Das erhaltene Rohprodukt wird abfiltriert und jeweils zweimal mit n-Hexan und Toluol heiß extrahiert und bei 30°C filtriert.

Ausbeute : 253 g
Schmelzpunkt : 205-208°C

Beispiel A.4

Herstellung des Diphenols der Formel (Ib), ($R^1$, $R^2$ = $CH_3$)

In einer Rührapparatur mit Rührer, Thermometer, Rückflußkühler und Gaseinleitungsrohr werden 2196 g (18 Mol) 2,6-Dimethylphenol, 38,2 g (0,36 Mol) β-Mercaptopropionsäure und 420 g (3 Mol) Dihydroisophoron (3,3,5-Trimethyl-cyclohexan-1-on) bei 35°C vorgelegt. In diese Lösung wird bei 35°C 5 h trockenes HCl-Gas eingeleitet. Man läßt ca. 10 h bei 30-35°C nachreagieren. Nach 95%igem Umatz des Ketons (GC-Kontrolle) gibt man zum Reaktionsgemisch 2,5 l Wasser und stellt durch Zugabe von 45%iger Na-OH-Lösung einen pH-Wert von 6 ein. Das Reaktionsgemisch wird eine Stunde bei 80°C gerührt und anschließend auf Raumtemperatur abgekühlt. Die wäßrige Phase wird abdekantiert und der verbleibende Rückstand mit Wasser bei 60°C gewaschen. Das erhaltene Rohprodukt wird abfiltriert und dreimal mit n-Hexan heiß extrahiert und filtriert.

Ausbeute : 856 g
Schmelzpunkt : 236-238°C.

Beispiel A.5

Herstellung des Diphenols der Formel (III)

Analog zu Beispiel A2 werden anstelle von 3 Mol Dihydroisophoron 3 Mol 3,3-Dimethylcyclohexanon eingesetzt. Das Produkt wies einen Schmelzpunkt von 199-201°C auf.

Die erfindungsgemäßen Diphenole der Formel (I) sind insbesondere geeignet zur Herstellung von hochmolekularen, thermoplastischen Polycarbonaten, die sich durch hohe Wärmeformbeständigkeit in Kombination mit einem guten sonstigen Eigenschaftsbild auszeichnen.

Gegenstand der vorliegenden Erfindung ist somit auch die Verwendung der Diphenole der Formel (I) zur Herstellung von hochmolekularen thermoplastischen, aromatischen Polycarbonaten.

Es können sowohl ein Diphenol der Formel (I) unter Bildung von Homopolycarbonaten als auch mehrere Diphenole der Formel (I) unter Bildung von Copolycarbonaten verwendet werden.

Außerdem können die Diphenole der Formel (I) auch im Gemisch mit anderen Diphenolen, beispielsweise mit denen der Formel HO-Z-OH (VII), zur Herstellung von hochmolekularen, thermoplastischen, aromatischen Polycarbonaten verwendet werden.

Geeignete andere Diphenole der Formel HO-Z-OH (VII) sind solche, in denen Z ein aromatischer Rest mit 6 bis 30 C-Atomen ist, der einen oder mehrere aromatische Kerne enthalten kann, substituiert sein kann und aliphatische Reste oder andere cycloaliphatische Reste als die der Formel (I) oder Heteroatome als Brückenglieder enthalten kann.

Beispiele für Diphenole der Formel (VII) sind
Hydrochinon,
Resorcin,
Dihydroxydiphenyle,
Bis-(hydroxyphenyl)-alkane,
Bis-(hydroxyphenyl)-cycloalkane,
Bis-(hydroxyphenyl)-sulfide,
Bis-(hydroxyphenyl)-ether,
Bis-(hydroxyphenyl)-ketone,
Bis-(hydroxyphenyl)-sulfone,
Bis-(hydroxyphenyl)-sulfoxide,
$\alpha,\alpha'$-Bis(hydroxyphenyl)-diisopropylbenzole
sowie deren kernalkylierte und kernhalogenierte Verbindungen.

Diese und weitere geeignete andere Diphenole sind z.B. in den US-PS 3028365, 2999835, 3148172, 3275601, 2991273, 3271367, 3062781, 2970131 und 2999846, in den deutschen Offenlegungsschriften 1570703, 2063050, 2063052, 22110956, der französischen Patentschrift 1561518 und in der Monographie "H. Schnell, Chemistry and Physics of Polycarbonates, Interscience Publishers, New York 1964", beschrieben.

Bevorzugte andere Diphenole sind beispielsweise :
4,4'-Dihydroxydiphenyl,
2,2-Bis-(4-hydroxyphenyl)-propan,
2,4-Bis-(4-hydroxyphenyl)-2-methylbutan,
1,1-Bis-(4-hydroxyphenyl)-cyclohexan,
$\alpha,\alpha'$-Bis-(4-hydroxyphenyl)-p-diisopropylbenzol,
2,2-Bis-(3-methyl-4-hydroxyphenyl)-propan,
2,2-Bis-(3-chlor-4-hydroxyphenyl)-propan,
Bis-(3,5-dimethyl-4-hydroxyphenyl)-methan,
2,2-Bis-(3,5-dimethyl-4-hydroxyphenyl)-propan,
Bis-(3,5-dimethyl-4-hydroxyphenyl)-sulfon,
2,4-Bis-(3,5-dimethyl-4-hydroxyphenyl)-2-methylbutan,
1,1-Bis-(3,5-dimethyl-4-hydroxyphenyl)-cyclohexan,
$\alpha,\alpha'$-Bis-(3,5-dimethyl-4-hydroxyphenyl)-p-diisopropylbenzol,
2,2-Bis-(3,5-dichlor-4-hydroxyphenyl)-propan und
2,2-Bis-(3,5-dibrom-4-hydroxyphenyl)-propan.

Besonders bevorzugte Diphenole der Formel (VII) sind beispielsweise :
2,2-Bis-(4-hydroxyphenyl)-propan,
2,2-Bis-(3,5-dimethyl-4-hydroxyphenyl)-propan,
2,2-Bis-(3,5-dichlor-4-hydroxyphenyl)-propan,
2,2-Bis-(3,5-dibrom-4-hydroxyphenyl)-propan und
1,1-Bis-(4-hydroxyphenyl)-cyclohexan.

Insbesondere ist 2,2-Bis-(4-hydroxyphenyl)-propan bevorzugt.

Die anderen Diphenole können sowohl einzeln als auch im Gemisch eingesetzt werden.

Das molare Verhältnis von erfindungsgemäß zu verwendenden Diphenolen der Formel (I) zu den gegebenenfalls mitzuverwendenden anderen Diphenolen, beispielsweise denen der Formel (VII), ist 100 Mol-% (I) zu 0 Mol-% anderem Diphenol bis 2 Mol-% (I) zu 98 Mol-% anderem Diphenol, vorzugsweise 100 Mol% (I) zu 0 Mol% anderem Diphenol bis 5 Mol% (I) zu 95 Mol% anderem Diphenol und insbesondere 100 Mol% (I) zu 0 Mol% anderem Diphenol bis 10 Mol% (I) zu 90 Mol% anderem Diphenol und ganz besonders 100 Mol% (I) zu 0 Mol% anderem Diphenol bis 20 Mol% (I) zu 80 Mol% anderem Diphenol.

Die hochmolekularen Polycarbonate aus den erfindungsgemäßen Diphenolen der Formel (I), gegebenenfalls in Kombination mit anderen Diphenolen können nach den bekannten Polycarbonatherstellungsverfahren hergestellt werden. Dabei können die verschiedenen Diphenole sowohl statistisch als auch blockweise miteinander verknüpft sein.

Gegenstand der vorliegenden Erfindung ist somit auch ein Verfahren zur Herstellung von hochmolekularen thermoplastischen, aromatischen Polycarbonaten aus Diphenolen, gegebenenfalls Kettenabbrechern und gegebenenfalls Verzweigern nach den bekannten Methoden der Polycarbonatherstellung, vorzugsweise durch Zweiphasengrenzflächen-Polykondensation, das dadurch gekennzeichnet ist, daß man als Diphenole solche der Formel (I) in Mengen von
100 Mol-% bis 2 Mol-%, vorzugsweise in Mengen von
100 Mol-% bis 5 Mol-% und insbesondere in Mengen von
100 Mol-% bis 10 Mol-%, und ganz besonders
100 Mol-% bis 20 Mol-%, bezogen jeweils auf die Gesamtmolmenge an eingesetzten Diphenolen, verwendet.

Als Verzweiger dienen, falls benutzt, in bekannter Weise geringe Mengen, vorzugsweise Mengen zwischen 0,05 und 2,0 Mol% (bezogen auf eingesetzte Diphenole), an drei- oder mehr als dreifunktionellen Verbindungen, insbesondere solchen mit drei oder mehr als drei phenolischen Hydroxylgruppen. Einige der verwendbaren Verbindungen mit drei oder mehr als drei phenolischen Hydroxylgruppen sind
Phloroglucin,
4,6-Dimethyl-2,4,6-tri-(4-hydroxyphenyl)-hepten-2,
4,6-Dimethyl-2,4,6-tri-(4-hydroxyphenyl)-heptan,
1,3,5-Tri-(4-hydroxyphenyl)-benzol,
1,1,1-Tri-(4-hydroxyphenyl)-ethan,
Tri-(4-hydroxyphenyl)-phenylmethan,
2,2-Bis-(4,4-bis-(4-hydroxyphenyl)-cyclohexyl)-propan,
2,4-Bis-(4-hydroxyphenyl-isopropyl)-phenol,
2,6-Bis-(2-hydroxy-5'-methyl-benzyl)-4-methylphenol,
2-(4-Hydroxyphenyl)-2-(2,4-dihydroxyphenyl)-propan,
Hexa-(4-(4-hydroxyphenyl-isopropyl)-phenyl)-ortho-terephthalsäureester,
Tetra-(4-hydroxyphenyl)-methan,
Tetra-(4-(4-hydroxyphenyl-isopropyl)-phenoxy)-methan und
1,4-Bis-((4'-,4"-dihydroxytriphenyl)-methyl)-benzol.

Einige der sonstigen dreifunktionellen Verbindungen sind 2,4-Dihydroxybenzoesäure, Trimesinsäure, Cyanurchlorid und 3,3-Bis-(3-methyl-4-hydroxyphenyl)-2-oxo-2,3-dihydroindol.

Als Kettenabbrecher zur Regelung des Molekulargewichtes dienen in bekannter Weise monofunktionelle Verbindungen in üblichen Konzentrationen. Geeignete Verbindungen sind z.B. Phenol, tert.-Butylphenole oder andere Alkyl-$C_1$-$C_7$-substituierte Phenole. Zur Regelung des Molekulargewichtes sind insbesondere kleine Mengen Phenole der Formel (VIII) geeignet

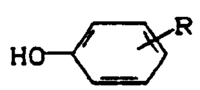

(VIII)

worin R einen verzweigten $C_8$- und/oder $C_9$-Alkylrest darstellt. Bevorzugt ist im Alkylrest R der Anteil Protonen in $CH_3$-Gruppen 47 bis 89% und der Anteil der Protonen in CH- und $CH_2$-Gruppen 53 bis 11% ; ebenfalls bevorzugt ist R in o- und/oder p-Stellung zur OH-Gruppe, und besonders bevorzugt die obere Grenze des ortho-Anteils 20%. Die Kettenabbrecher werden im allgemeinen in Mengen von 0,5 bis 10, bevorzugt 1,5 bis 8 Mol-%, bezogen auf Diphenole, eingesetzt.

Die erfindungsgemäßen Polycarbonate können vorzugsweise durch Phasengrenzflächenpolykondensation (vgl. H. Schnell, "Chemistry and Physics of Polycarbonates", Polymer Reviews, Vol. IX, Seite 33 ff., Interscience Publ., 1964) in an sich bekannter Weise hergestellt werden. Hierbei werden die Diphenole der Formel (I) in wäßrig alkalischer Phase gelöst. Zur Herstellung von Co-Polycarbonaten mit anderen Diphenolen werden Gemische von Diphenolen der Formel (I) und den anderen Diphenolen, beispielsweise denen der Formel (VII), eingesetzt. Zur Regulierung des Molekulargewichtes können Kettenabbrecher z.B. der Formel (VIII) zugegeben werden. Dann wird in Gegenwart einer inerten, vorzugsweise Polycarbonat lösenden, organischen Phase mit Phosgen nach der Methode der Phasengrenzflächenkondensation umgesetzt. Die Reaktionstemperatur liegt zwischen 0°C und 40°C.

Die gegebenenfalls mitzuverwendenden 0,05 bis 2 Mol-% an Verzweigern können entweder mit den Diphenolen in der wäßrig alkalischen Phase vorgelegt werden oder in dem organischen Lösungsmittel gelöst vor der Phosgenierung zugegeben werden.

Neben den Diphenolen der Formel (I) sowie den anderen Diphenolen (VII) können auch deren Mono-und/ oder Bischlorkohlensäureester verwendet werden, wobei diese in organischen Lösungsmitteln gelöst zugegeben werden. Die Menge an Kettenabbrechern sowie an Verzweigern richtet sich dann nach Molen Diphenolat-Struktureinheiten von (I) und gegebenenfalls von den anderen Di-phenolen wie beispielsweise von (VII) ; ebenso kann bei Einsatz von Chlorkohlensäureestern die Phosgenmenge in bekannter Weise entsprechend reduziert werden.

Geeignete organische Lösungsmittel zum Auflösen der Kettenabbrecher sowie gegebenenfalls der Verzweiger und der Chlorkohlensäureester sind beispielsweise Methylenchlorid, Chlorbenzol, Aceton, Acetonitril sowie Mischungen dieser Lösungsmittel, insbesondere Mischungen aus Methylenchlorid und Chlorbenzol. Gegebenenfalls können die verwendeten Kettenabbrecher und Verzweiger im gleichen Solvens gelöst werden.

Als organische Phase für die Phasengrenzflächenpolykondensation dient beispielsweise Methylenchlorid, Chlorbenzol sowie Mischungen aus Methylenchlorid und Chlorbenzol.

Als wäßrige alkalische Phase dient beispielsweise wäßrige NaOH-Lösung.

Die Bildung der erfindungsgemäßen Polycarbonate durch Phasengrenzflächenpolykondensation kann in üblicher Weise durch Katalysatoren wie tertiäre Amine, insbesondere tertiäre aliphatische Amine wie Tributylamin oder Triethylamin beschleunigt werden ; die Katalysatoren können in Mengen von 0,05 bis 10 Mol-%, bezogen auf Mole an eingesetzten Diphenolen eingesetzt werden. Die Katalysatoren können vor Beginn der Phosgenierung oder während oder auch nach der Phosgenierung zugesetzt werden.

Die erfindungsgemäßen Polycarbonate können in bekannter Weise abgetrennt werden.

Die erfindungsgemäßen hochmolekularen, thermoplastischen, aromatischen Polycarbonate können auch nach dem bekannten Verfahren in homogener Phase, dem sogenannten "Pyridinverfahren" sowie nach dem bekannten Schmelzumesterungsverfahren unter Verwendung von beispielsweise Diphenylcarbonat anstelle von Phosgen hergestellt werden. Auch hier werden die erfindungsgemäßen Polycarbonate in bekannter Weise isoliert.

Die nach dem erfindungsgemäßen Verfahren erhältlichen Polycarbonate haben bevorzugt Molekulargewichte Mw (Gewichtsmittel, ermittelt durch Gelchromatographie nach vorheriger Eichung) von mindestens 10.000 g/mol, besonders bevorzugt von 10.000 bis 300.000 g/mol. Soweit die erfindungsgemäßen Polycarbonate als Spritzgußmaterial Verwendung finden, sind besonders bevorzugte Molekulargewichte zwischen 20.000 und 80.000 g/mol. Soweit die erfindungsgemäßen Polycarbonate als Gießfolien Verwendung finden, sind insbesondere Molekulargewichte Mw zwischen 100.000 und 250.000 g/mol bevorzugt. Für die Herstellung von Extrusionsfolien sind erfindungsgemäße Polycarbonate mit Mw zwischen 25.000 und 150.000 g/mol bevorzugt. Die erfindungsgemäßen Polycarbonate können linear oder verzweigt sein, sie sind Homopolycarbonate oder Copolycarbonate auf Basis der Diphenole der Formel (I).

Gegenstand der vorliegenden Erfindung sind somit auch hochmolekulare thermoplastische, aromatische Polycarbonate mit $\overline{M}w$ (Gewichtsmittelmolekulargewichten) von mindestens 10000, vorzugsweise von 10000 bis 300000 und, für Anwendungen im Spritzgußbereich, insbesondere von 20000 bis 80000, erhältlich nach dem erfindungsgemäßen Verfahren aus Diphenolen der Formel (I), die linear oder verzweigt sind.

Gegenstand der vorliegenden Erfindung sind somit auch hochmolekulare, thermoplastische, aromatische Polycarbonate mit $\overline{M}w$ (Gewichtsmittelmolekulargewichten) von mindestens 10000, vorzugsweise von 10000 bis 300000 g/mol und, für Anwendungen im Spritzgußbereich, insbesondere von 20000 bis 80000, die bifunktionelle Carbonatstruktureinheiten der Formel (Ia)

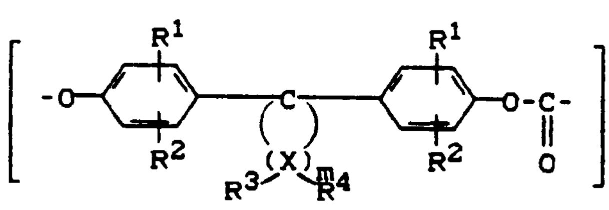

(Ia)

worin

X, $R^1$, $R^2$, $R^3$, $R^4$ und m die für die Formel (I) genannte Bedeutung haben,

in Mengen von 100 Mol-% bis 2 Mol-%, vorzugsweise in Mengen von 100 Mol-% bis 5 Mol-% und insbesondere in Mengen von 100 Mol-% bis 10 Mol-% und ganz besonders 100 Mol-% bis 20 Mol-%, bezogen jeweils auf die Gesamtmenge von 100 Mol-% an difunktionellen Carbonatstruktureinheiten im Polycarbonat enthalten.

Die erfindungsgemäßen Polycarbonate enthalten somit jeweils zu 100 Mol-% komplementäre Mengen an

anderen difunktionellen Carbonatstukturelnheiten, beispielsweise solchen der Formel (VIIa)

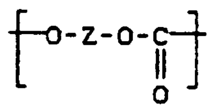

(VIIa),

also 0 Mol-% (einschließlich) bis 98 Mol-% einschließlich, vorzugsweise 0 Mol-% bis 95 Mol-% und insbesondere 0 Mol-% bis 90 Mol-% und ganz besonders bevorzugt 0 Mol-% bis 80 Mol-%, bezogen jeweils auf die Gesamtmenge (von 100 Mol-%) an difunktionellen Carbonatstrukturelnheiten im Polycarbonat.

Polycarbonate auf Basis von cycloaliphatischen Bisphenolen sind grundsätzlich bekannt und z.B. in EP-A 164476, EP-A 166834, DE-OS 3345945, DE-OS 2063052, FR-PS 1427998, WO 8000348, BE-PS 785189 beschrieben. Sie haben häufig relativ hohe Einfriertemperaturen, aber andere, wichtige physikalische Eigenschaften wie UV- und Wärmealterungsstabilität sind unzureichend.

Es hat sich nun überraschenderweise gezeigt, daß, wie bereits erwähnt, durch den Einbau der erfindungsgemäßen Diphenole der Formel (I) neue Polycarbonate mit hoher Wärmeformbeständigkeit erhalten werden, die auch sonst ein gutes Eigenschaftsbild haben. Dies gilt ganz besonders für die Polycarbonate auf Basis der Diphenole (Ib)

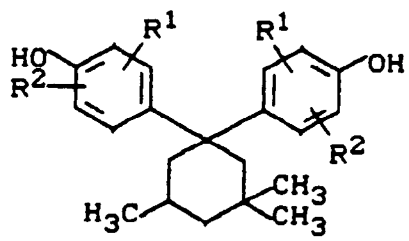

(Ib),

worin
$R^1$ und $R^2$ unabhängig voneinander die für Formel (I) genannte Bedeutung haben und besonders bevorzugt Wasserstoff sind.

Somit sind Gegenstand der Erfindung vorzugsweise Polycarbonate, in denen in den Struktureinheiten der Formel (Ia) m = 4 oder 5 und ganz besonders solche mit Struktureinheiten der Formel (Ic)

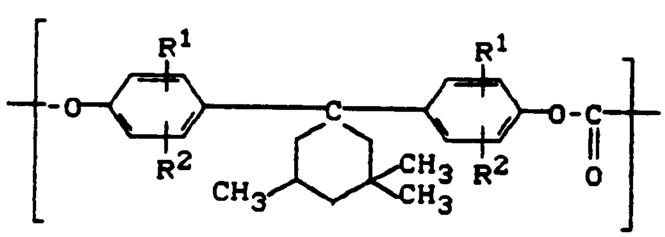

(Ic)

worin
$R^1$ und $R^2$ die für Formel (Ia) genannte Bedeutung haben, besonders bevorzugt aber Wasserstoff sind.

Diese Polycarbonate auf Basis der Diphenole der Formel (Ib), worin insbesondere $R^1$ und $R^2$ Wasserstoff sind, besitzen zur hohen Wärmeformbeständigkeit außerdem eine gute UV-Stabilität und ein gutes Fließverhalten in der Schmelze, was nicht zu erwarten war.

Durch die freie Kombinierbarkeit mit anderen Diphenolen, insbesondere mit denen der Formel (VII) lassen sich zudem die Polycarbonateigenschaften in günstiger Weise variieren.

Die Isolierung der nach dem erfindungsgemäßen Verfahren erhältlichen Polycarbonate geschieht in bekannter Weise, indem man die bei Phasengrenzflächenverfahren erhaltene organische Phase abtrennt, neutral und elektrolytfrei wäscht und dann beispielsweise über einen Eindampfextruder als Granulat isoliert.

Den erfindungsgemäßen Polycarbonaten können noch vor oder nach ihrer Verarbeitung die für thermoplastische Polycarbonate üblichen Additive wie Stabilisatoren, Entformungsmittel, Pigmente, Flammschutzmittel, Antistatika, Füllstoffe und Verstärkungsstoffe in den üblichen Mengen zugesetzt werden.

Im einzelnen können beispielsweise Ruß, Graphit, Kieselgur, Kaolin, Tone, $CaF_2$, $CaCO_3$, Aluminiumoxide, Glas fasern, Bariumsulsat und anorganische Pigmente sowohl als Füllstoffe als auch als Nucleierungsmittel zugesetzt werden, sowie als Entformungsmittel beispielsweise Glycerinstearate, Pentaerythrittetrastearat und Trimethylolpropantristearat.

Die erfindungsgemäßen Polycarbonate können zu Formkörpern verarbeitet werden, indem man beispielsweise die in bekannter Weise isolierten Polycarbonate zu Granulat extrudiert und dieses Granulat gegebenenfalls nach Zusatz der obengenannten Additive durch Spritzguß zu verschiedenen Artikeln in bekannter Weise verarbeitet.

Die erfindungsgemäßen Polycarbonate sind als Formkörper überall dort einsetzbar, wo die bislang bekannten Polycarbonate eingesetzt werden, also im Elektrosektor sowie im Bausektor für Abdeckungen und Verglasungen, und zwar dann, wenn erhöhte Wärmeformbeständigkeit bei gleichzeitig guter Verarbeitbarkeit, also wenn komplizierte Bauteile hoher Wärmeformbeständigkeit verlangt werden.

Weitere Verwendungen der erfindungsgemäßen Polycarbonate :

A.

Als optische Datenspeicher, beispielsweise Compact Discs :

Die Herstellung solcher Datenspeicher ist bekannt (siehe beispielsweise J. Hennig, Vortrag auf der Tagung "Neue Polymere" Bad Nauheim 14/15.04.1986 "Polymere als Substrate für optische Plattenspeicher" oder Philips techn. Rev. 33, 178-180, 1973, No. 7 und 33, 186-189, 1973 Nol 7).

B.

Zur Herstellung von Sicherheitsscheiben.

Sicherheitsscheiben haben meist eine Dicke von 2 mm bis 10 mm und können mit $SiO_x$, worin x einen Wert von 1 bis 2 hat, bedampft sein oder in Verbindung mit Glasscheiben eingesetzt sein. Sicherheitsscheiben sind bekanntlich in vielen Bereichen von Gebäuden, Fahrzeugen und Flugzeugen erforderlich sowie als Schilde und Helme von Nutzen.

C.

Als Lackrohstoffe.

D.

Zur Herstellung von Blaskörpern (siehe beispielsweise US-Patent 2964794).

E.

Zur Herstellung von lichtdurchlässigen Platten, insbesondere von Hohlkammerplatten, beispielsweise zum Abdecken von Gebäuden wie Bahnhöfen, Gewächshäusern und Beleuchtungsanlagen.

F.

Zur Herstellung von Schaumstoffen. (Siehe beispielsweise DE-AS 1031507).

G.

Zur Herstellung von Fäden und Drähten. [Siehe beispielsweise DE-AS 1137167 und DE-OS 1785137).

H.

Als transluzente Kunststoffe mit einem Gehalt an Glasfasern für lichttechnische Zwecke. (Siehe beispielsweise DE-OS 1544020).

I.

Zur Herstellung von Präzisionsspritzgußteilchen, wie beispielsweise Linsenhalterungen. Hierzu verwendet

man Polycarbonate mit einem Gehalt an Glasfasern, die gegebenenfalls zusätzlich etwa 1 Gew.-% bis 10 Gew.-% $MoS_2$, bezogen auf Gesamtgewicht, enthalten.

K.

Zur Herstellung optischer Geräteteile, insbesondere Linsen für Photo- und Filmkameras. (Siehe beispielsweise DE-OS 2701173).

L.

Als Lichtübertragungsträger, insbesondere als Lichtleitkabel. (Siehe beispielsweise EP-OS 0089801).

M.

Als Elektroisolierstoffe für elektrische Leiter.

O.

Als Trägermaterial für organische Fotoleiter.

P.

Zur Herstellung von Leuchten, z.B. Scheinwerferlampen, als sogenannte "head-lamps" oder Streulichtscheiben.

Insbesondere können aus den hochmolekularen aromatischen Polycarbonaten der Erfindung Folien hergestellt werden. Die Folien haben bevorzugte Dicken zwischen 1 und 1500 µm, insbesondere bevorzugte Dicken zwischen 10 und 900 µm.

Die erhaltenen Folien können in an sich bekannter Weise monoaxial oder biaxial gereckt werden, bevorzugt im Verhältnis 1 : 1.5 bis 1 : 3.

Die Folien können nach den bekannten Verfahren zur Folienerzeugung hergestellt werden, z.B. durch Extrusion einer Polymerschmelze durch eine Breitschlitzdüse, durch Blasen auf einer Folienblasmaschine, Tiefziehen oder Gießen. Dabei vergießt man eine konzentrierte Lösung des Polymeren in einem geeigneten Lösungsmittel auf eine ebene Unterlage, verdampft das Lösungsmittel und hebt die gebildete Folie von der Unterlage ab.

Die Folien können in an sich bekannter Weise bei Temperaturen zwischen Zimmertemperatur und einer Temperatur, bei der die Viskosität der Polymerschmelze noch nicht zu stark erniedrigt ist, im allgemeinen bis zu etwa 370°C, auf bekannten Vorrichtungen verstreckt werden.

Die Folienherstellung durch Gießen der Polycarbonatlösungen erfolgt beispielsweise, indem man konzentrierte Lösungen des Polycarbonats in einem geeigneten Lösungsmittel auf ebene Oberflächen ausgießt und bei einer Temperaturführung der ebenen Oberflächen zwischen Raumtemperatur und 150°C das Lösungsmittel anschließend verdampft. Man kann die konzentrierten Lösungen der Polycarbonate auch auf Flüssigkeiten aufbringen, welche eine höhere Dichte als die der konzentrierten Lösungen haben, nicht mit dem verwendeten Lösungsmittel verträglich sind und das Polycarbonat nicht lösen, und nach dem Spreiten die Folien durch Verdampfen des für das Polycarbonat verwendeten Lösungsmittels und gegebenenfalls auch der Flüssigkeit mit höherer Dichte gewinnen.

Die erfindungsgemäßen Folien haben eine besonders hohe Wärmeformbeständigkeit und sind für viele Gase bei dennoch guter Selektivität durchlässig. Sie können daher vorteilhaft als Membrane für die Gaspermeation verwendet werden.

Dabei ist es möglich, diese Folien für sich allein zu verwenden.

Man kann mit ihnen natürlich auch Verbundfolien mit anderen Kunststoff-Folien herstellen, wobei prinzipiell, je nach gewünschter Anwendung und Endeigenschaft der Verbundfolie, alle bekannten Folien als Partner in Frage kommen. Zum Beispiel kann man einen Verbund aus zwei oder mehr Folien erzeugen, indem man die Einzelfolien, darunter die erfindungsgemäße Polycarbonatfolie, aufeinanderlegt und bei geeigneten Temperaturen, die von den Erweichungspunkten der einzelnen Folien bestimmt werden, unter Anwendung von Druck verpreßt. Man kann auch das bekannte Folien-Coextrusionsverfahren anwenden.

Die Herstellung der Verbundfolien erfolgt, indem man zunächst in bekannter bzw. in der oben beschriebenen Weise in optimaler Temperaturführung die Folien der Einzelkomponenten herstellt. Anschließend bringt man die nicht abgekühlten Folien ohne größere Verstreckung auf eine gemeinsame Temperatur, die vorzugsweise zwischen Zimmertemperatur und 370°C liegt. Die Folien werden dann über Walzen zusammengeführt und kurzzeitig verpreßt. Dabei kann ein Druck zwischen 2 und 500 bar angewendet werden. Das Verfahren kann auch mit mehr als einer anderen Folie als der aus den Polycarbonaten durchgeführt werden, wobei beispielsweise zunächst jeweils die anderen Folien in der bisher bekannten Weise zusammengebracht werden und danach mit der Folie aus den Polycarbonaten unter dem oben beschriebenen Druck verpreßt werden.

Die Folien oder Verbundfolien können in bekannter Weise darüber hinaus als homogene Membrane, Kompositionsmembrane oder asymmetrische Membrane hergestellt werden bzw. verwendet werden. Die Membranen, Folien oder Verbundfolien können flach ein, Hohlkörper verschiedener Geometrien bilden — zylinderförmig, kugelförmig, schlauchförmig — oder auch Hohlfasern sein. Solche Formteile können nach den dem Fachmann bekannten Verfahren hergestellt werden.

Als Polymere, aus denen sich für die Herstellung von Verbundfolien mit den erfindungsgemäßen Polycarbonat-Folien Folien herstellen lassen, kommen je nach Anwendung verschiedene Polymere in Frage, die im folgenden aufgeführt werden. Je nach Anwendung kann man somit auch gasundurchlässige Verbundfolien erreichen, die gegenüber dem Stand der Technik eine bessere Wärmeformbeständigkeit haben, oder man kann wärmeformbeständige, gasdurchlässige Verbundfolien erhalten — je nach Wahl der Verbundpartner.

Im folgenden sind Materialien angegeben, die mit den erfindungsgemäßen Folien kombinierbare Folien liefern. Diese Materialien sind als Komponenten (b) bezeichnet.

Als Komponente (b) geeignete Thermoplasten sind

b1) amorphe Thermoplaste, vorzugsweise solche mit einer Glastemperatur von mehr als 40°C, insbesondere von 60°C bis 220°C, und

b2) teilkristalline Thermoplaste, vorzugsweise solche mit einer Schmelztemperatur von mehr als 60°C, insbesondere von 80°C bis 400°C.

Elastomere für die Komponente b) sind

b3) solche, die eine Glastemperatur von unter 0°C, vorzugsweise von unter – 10°C und insbesondere von – 15°C bis – 140°C, haben.

Beispiele für amorphe Thermoplasten b1) sind amorphe Polymere aus der Klasse der Polycarbonate, Polyamide, Polyolefine, Polysulfone, Polyketone, thermoplastische Vinylpolymerisate wie Polymethylacrylsäureester oder Homopolymerisate von Vinylaromaten, Copolymerisate von Vinylaromaten oder Pfropfpolymerisate von Vinylmonomeren auf Kautschuke, Polyether, Polyimide, thermoplastische Polyurethane, aromatische Polyester(carbonate) und flüssigkristalline Polymere.

Beispiele für kristalline Thermoplasten b2) sind aliphatische Polyester, Polyarylensulfide sowie die teilkristallinen Vertreter der vorstehend unter b1) subsummierten Thermoplasten.

Beispiele für Elastomere b3) sind die verschiedensten Kautschuke wie Ethylen-Propylen-Kautschuk, Polyisopren, Polychloropren, Polysiloxane, ataktisches Polypropylen, Dien-, Olefin- und Acrylatkautschuke und Naturkautschuke, Styrol-Butadien-Blockcopolymere, Ethylen-Copolymerisate mit Vinylacetate oder mit (Meth)acrylsäureestern, elastische Polyurethane soweit nicht als Thermoplasten unter b1) oder b2) subsumiert und elastische Polycarbonat-Polyether-Blockcopolymere.

Amorphe Thermoplasten b1) sind insbesondere Polycarbonate (außer den erfindungsgemäßen Polycarbonaten). Polycarbonate können sowohl Homopolycarbonate als auch Copolycarbonate sein, sie können sowohl linear als auch verzweigt sein. Besonders bevorzugtes Bisphenol für die Polycarbonate ist Bisphenol-A.

Die Molekulargewichte $\overline{M}w$ (Gewichtsmittelmolekulargewicht, ermittelt nach der Gelpermeationschromatographie in Tetrahydrofuran) der thermoplastischen Polycarbonate liegen zwischen 10.000 und 300.000, vorzugsweise zwichen 12.000 und 150.000.

Die thermoplastischen Polycarbonate sind sowohl einzeln als auch in Mischungen als Komponente b) verwendbar.

Bevorzugte andere Thermoplasten sind auch aliphatische, thermoplastische Polyester, besonders bevorzugt Polyalkylenterephthalate, beispielsweise solche auf Basis von Ethylenglykol, Propandiol-1,3, Butandiol-1,4, Hexandiol-1,6 und 1,4-Bis-hydroxymethylcyclohexan.

Die Molekulargewichte ($\overline{M}w$) dieser Polyalkylenterephthalate liegen zwischen 10.000 und 80.000. Die Polyalkylenterephthalate können nach bekannten Verfahren beispielsweise aus Terephthalsäuredialkylester und dem entsprechenden Diol durch Umesterung erhalten werden (s. z.B. US-Patente 2647885, 2643989, 2534028, 2578660, 2742494, 2901466).

Weitere bevorzugte andere Thermoplasten sind thermoplastische Polyamide.

Es eignen sich alle teilkristallinen Polyamide, insbesondere Polyamid-6, Polyamid-6,6 und teilkristalline

Copolyamide auf Basis dieser beiden Komponenten. Weiterhin kommen teilkristalline Polyamide in Betracht, deren Säurekomponente insbesondere ganz oder teilweise Adipinsäure beziehungsweise Caprolactam aus Terephthalsäure und/oder Isophthalsäure und/oder Korksäure und/oder Sebacinsäure und/oder Azelainsäure und/oder Dodecandicarbonsäure und/oder Adipinsäure und/oder einer Cyclohexandicarbonsäure besteht, und deren Diaminkomponente ganz oder teilweise insbesondere aus m- und/oder p-Xylylendiamin und/oder Hexamethylendiamin und/oder 2,2,4- und/oder 2,4,4-Trimethylhexamethylendiamin und/oder Isophorondiamin und/oder 1,4-Diaminobutan bestehen und deren Zusammensetzungen im Prinzip aus dem Stand der Technik bekannt sind (siehe beispielsweise Encyclopedie of Polymers, Vol. 11, S. 315 ff).

Außerdem sind geeignet teilkristalline Polyamide, die ganz oder teilweise aus Lactamen mit 6 bis 12 C-Atomen, gegebenenfalls unter Mitverwendung einer oder mehrerer der obengenannten Ausgangskomponenten, hergestellt werden.

Besonders bevorzugte teilkristalline Polyamide sind Polyamid-6 und Polyamid-6,6 oder Copolyamide mit geringem Anteil, bis etwa 10 Gewichtsprozent an anderen Komponenten.

Geeignete Polyamide sind auch amorphe Polyamide, erhalten beispielsweise durch Polykondensation von Diaminen, wie beispielsweise von Hexamethylendiaminen, Decamethylendiamin, 2,2,4- beziehungsweise 2,4,4-Trimethylhexamethylendiamin, m- beziehungsweise p-Xylylendiamin, Bis-(4-aminocyclohexyl)-methan, Gemischen aus 4,4′- und 2,2′-Diaminodicyclohexylmethan, 2,2-Bis-(4-aminocyclohexyl)-propan, 3,3′-Dimethyl-4,4′-diamino-dicyclohexylmethan, 3-Aminoethyl-3,5,5-trimethyl-cyclohexylamin, 2,5-Bis-(aminomethyl)-norbornan, 2,6-Bis-(aminomethyl)-norbornan, 1,4-Diamino-methylcyclohexan und von beliebigen Gemischen dieser Diamine, mit Dicarbonsäuren wie beispielsweise mit Oxalsäure, Adipinsäure, Azelainsäure, Decandicarbonsäure, Heptadecandicarbonsäure, 2,2,4-Trimethyladipinsäure, 2,4,4-Trimethyladipinsäure, Isophthalsäure und Terephthalsäure und mit beliebigen Gemischen dieser Dicarbonsäuren. Es sind somit auch amorphe Copolyamide einbezogen, die durch Polykondensation mehrerer der vorstehend genannten Diamine und/oder Dicarbonsäuren erhalten werden.

Ferner sind amorphe Copolyamide einbezogen, die unter Mitverwendung von ω-Aminocarbonsäuren wie ω-Aminocapronsäure, ω-Aminoundecansäure oder ω-Aminolaurinsäure oder von deren Lactamen hergestellt sind.

Besonders geeignete, amorphe, thermoplastische Polyamide sind solche, die aus Isophthalsäure, Hexamethylendiamin und weiteren Diaminen wie 4,4′-Diaminodicyclohexylmethan, Isophorondiamin, 2,2,4- beziehungsweise 2,4,4-Trimethylhexamethylendiamin, 2,5- und/oder 2,6-Bis-(aminomethyl)-norbornan erhältlich sind, solche, die aus Isophthalsäure, 4,4′-Diamino-di-cyclohexylmethan und ω-Caprolactam erhältlich sind, solche, die aus Isophthalsäure, 3,3-Dimethyl-4,4′-diamino-dicyclohexylmethan und ω-Laurinlactam erhältlich sind, und solche, die aus Terephthalsäure und dem Isomerengemisch aus 2,2,4- und 2,4,4-Trimethylhexamethylendiamin erhältlich sind.

Anstelle des reinen 4,4′-Diaminodicyclohexylmethans können auch Gemische der stellungsisomeren Diaminodicyclohexylmethane eingesetzt werden, die sich zusammensetzen aus
70 bis 99 Mol-% des 4,4′-Diaminoisomeren
1 bis 30 Mol-% des 2,4′-Diaminoisomeren
0 bis 2 Mol-% des 2,2′-Diaminoisomeren
und gegebenenfalls entsprechend höherkondensierten Diaminen, die durch Hydrierung von Diaminodiphenylmethan technischer Qualität erhalten werden.

Geeignete thermoplastische Polyamide können auch aus Mischungen von teilkristallinen und amorphen Polyamiden bestehen, wobei vorzugsweise der Anteil an amorphem Polyamid unter dem Anteil an teilkristallinem Polyamid liegt. Auch die amorphen Polyamide und deren Herstellung sind aus dem Stand der Technik bekannt (siehe beispielsweise Ullmann, Enzyklopädie d. technischen Chemie, Band 19, S. 50).

Bevorzugte andere Thermoplasten b) sind auch thermoplastische lineare oder verzweigte Polyarylensulfide. Sie haben Struktureinheiten der allgemeinen Formel

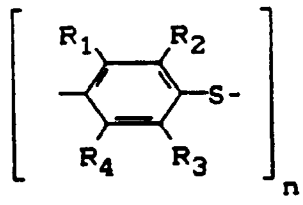

wobei $R_1$ bis $R_4$ gleich oder verschieden sein können und $C_1$-$C_6$-Alkyl, Phenyl oder Wasserstoff bedeuten. Die Polyarylensulfide können auch Diphenyl-Einheiten enthalten.

Polyarylensulfide und ihre Herstellung sind bekannt (siehe beispielsweise US-PS 3354129 und EP-A 0171021).

Weitere bevorzugte andere Thermoplasten b) sind thermoplastische Polyarylensulfone.

Geeignete Polyarylensulfone haben mittlere Gewichtsmittelmolekulargewichte $\overline{M}w$ (gemessen nach der Lichtstreumethode in $CHCl_3$) von 1.000 bis 200.000, vorzugsweise von 20.000 bis 60.000.

Beispiele sind die nach bekannten Verfahren erhältlichen Polyarylensulfone aus 4,4′-Dichlordiphenylsulfon und einem Bisphenol, insbesondere 2,2-Bis-(4-hydroxyphenyl)-propan, mit $\overline{M}w$ von 2.000 bis 200.000.

Polyarylensulfone sind bekannt (siehe beispielsweise US-PS 3264536, DE-AS 1794171, GB-PS 1264900, US-PS 3641207, EP-A-0038028, DE-OS 3601419 und DE-OS 3601420). Die Polyarylensulfone können auch in bekannter Weise verzweigt sein (siehe beispielsweise DE-OS 2305413).

Bevorzugte andere Thermoplasten b) sind auch bekannte thermoplastische Polyphenylenoxide, vorzugsweise Poly-(2,6-dialkyl-1,4-phenylenoxide) mit Molekulargewichten $\overline{M}w$ (Gewichtsmittel, gemessen durch Lichtstreuung in Chloroform) von 2.000 bis 100.000, vorzugsweise von 20.000 bis 60.000.

Sie können, wie bekannt, durch oxidierende Kondensation von 2,6-Dialkylphenolen mit Sauerstoff in Anwesenheit von Kupfersalzen und tertiären Aminen als Katalysator erhalten werden (siehe beispielsweise DE-OS 2126434 und US-PS 3306875).

Geeignet sind insbesondere Poly-[2,6-di($C_1$-$C_4$-alkyl)-1,4-phenylenoxide], z.B. Poly-(2,6-dimethyl-1,4-phenylenoxid).

Bevorzugte andere Thermoplasten b) sind auch aromatische Polyetherketone selbst (siehe beispielsweise GB-PS 1078234, US-PS 4010147 und EP-OS 0135938), außer denen auf Basis von Diphenolen der Formel I.

Sie enthalten das wiederkehrende Strukturelement

—O-E-O-E′—,

worin —E′— der zweibindige Rest eines Bisarylketons und —O-E-O— ein zweibindiger Diphenolat-Rest ist.

Sie können beispielsweise gemäß GB-PS 1078234 aus Dialkalidiphenolaten der Formel Alkali-O-E-O-Alkali und Bis-(halogenaryl)-ketonen der Formel Hal-E′-Hal (mit Hal = Halogen) hergestellt werden. Ein geeignetes Dialkalidiphenolat ist z.B. das des 2,2-Bis-(4-(hydroxyphenyl)-propans, ein geeignetes Bis-(halogenaryl)-keton ist das 4,4′-Dichlorbenzophenon.

Bevorzugte andere Thermoplasten b) sind auch thermoplastische Vinyl-Polymerisate.

Vinyl-Polymerisate im Sinne dieser Erfindung sind Homopolymerisate von Vinylverbindungen, Copolymerisate von Vinylverbindungen und Pfropfpolymerisate von Vinylverbindungen auf Kautschuke.

Erfindungsgemäß geeignete Homopolymerisate und Copolymerisate sind solche von Styrol, α-Methylstyrol, Acrylnitril, Methacrylnitril, $C_1$-$C_{12}$-(Cyclo)-Alkyl-Estern der (Meth)Acrylsäure, $C_1$-$C_4$-Carbonsäure-Vinylester, wobei die Copolymerisate aus Mischungen dieser Vinyl-Verbindungen nach bekannten Methoden erhältlich sind.

Die Homo- beziehungsweise Copolymerisate sollen Grenzviskositäten (Staudinger-Indices) zwischen 0,3 und 1,5 dl/g (gemessen bei 23°C in Toluol in bekannter Weise) haben.

Geeignete Vinylpolymerisate sind beispielsweise thermoplastische Poly-$C_1$-$C_4$-Alkylmethacrylate, beispielsweise solche des Methyl-, Ethyl-, Propyl- oder Butylmethacrylsäureesters, vorzugsweise des Methyl- oder Ethyl-methacrylsäureesters. Es sind sowohl Homopolymerisate als auch Copolymerisate dieser Methacrylsäureester darunter zu verstehen. Darüber hinaus können andere, ethylenisch ungesättigte copolymerisierbare Monomere wie beispielsweise (Meth)Acrylnitril, (α-Methyl)-Styrol, Bromstyrol, Vinylacetat, Acrylsäure-$C_1$-$C_8$-alkylester, (Meth)Acrylsäure, Ethylen, Propylen und N-Vinylpyrrolidon in untergeordneten Mengen einpolymerisiert sein.

Die erfindungsgemäß geeigneten thermoplastischen Poly-$C_1$-$C_4$-alkyl-methacrylate sind literaturbekannt oder nach literaturbekannten Verfahren erhältlich.

Geeignete Vinylpolymerisate sind auch Copolymerisate aus Styrol oder α-Methylstyrol und Acrylnitril, die gegebenenfalls bis zu 40 Gew.-% Ester der Acrylsäure oder Methacrylsäure, insbesondere Methylmethacrylat oder n-Butylacrylat enthalten. Styrolderivate müssen auf jeden Fall als Monomere enthalten sein. Die Styrolderivate sind dabei in Anteilen zwischen 100 und 10 Gew.-%, bevorzugt zwischen 90 bis 20 Gew.-%, besonders bevorzugt zwischen 80 bis 30 Gew.-%, enthalten, die nach üblichen Verfahren wie radikalische Polymerisation in Masse, Lösung, Suspension oder Emulsion, bevorzugt aber durch radikalische Emulsionspolymerisation in Wasser erhalten werden.

Geeignete Pfropfpolymerisate entstehen durch Polymerisation der oben genannten Vinylmonomeren oder Vinylmonomerengemische in Gegenwart von Kautschuken mit Glastemperaturen $< 0°C$, vorzugsweise $< -20°C$. Die Pfropfpolymerisate enthalten in der Regel 1 bis 85 Gew.-%, bevorzugt 10 bis 80 Gew.-%, Kautschuk. Die Pfropfpolymerisate lassen sich durch übliche Verfahren in Lösung, Masse oder Emulsion, bevorzugt in Emulsion, herstellen, wobei Vinylmonomerengemische simultan oder sukzessive pfropfpolymerisiert werden

können.

Geeignete Kautschuke sind vorzugsweise Dienkautschuke und Acrylatkautschuke.

Dienkautschuke sind beispielsweise Polybutadien, Polyisopren und Copolymerisate von Butadien mit bis zu 35 Gew.-% Comonomeren wie Styrol, Acrylnitril, Methylmethacrylat und $C_1$-$C_6$-Alkylacrylaten.

Acrylatkautschuke sind beispielsweise vernetzte, teilchenförmige Emulsionspolymerisate aus $C_1$-$C_6$-Alkylacrylaten, insbesondere $C_2$-$C_6$-Alkylacrylaten, gegebenenfalls im Gemisch mit bis zu 15 Gew.-% anderen, ungesättigten Monomeren wie Styrol, Methylmethacrylat, Butadien, Vinylmethylether, Acrylnitril, und aus wenigstens einem polyfunktionellem Vernetzer wie beispielsweise Divinylbenzol, Glykol-bis-acrylate, Bisacrylamide, Phosphorsäuretriallylester, Zitronensäuretriallylester, Allylester von Acrylsäure und Methacrylsäure, Triallylisocyanurat, wobei die Acrylatkautschuke bis zu 4 Gew.-% der vernetzenden Comonomere enthalten können.

Zur Herstellung der Pfropfpolymerisate sind auch Gemische von Dien- mit Acrylatkautschuken sowie Kautschuke mit einer Kern-Mantel-Struktur geeignet.

Die Kautschuke müssen zur Pfropfpolymerisation in Form diskreter Teile vorliegen, z.B. als Latex. Diese Teilchen haben i.a. mittlere Durchmesser von 10 nm bis 2.000 nm.

Die Pfropfpolymerisate können nach bekannten Verfahren, beispielsweise durch radikalische Emulsionspfropfpolymerisation der Vinylmonomeren in Gegenwart von Kautschuklatices bei Temperaturen von 50 bis 90°C unter Verwendung wasserlöslicher Initiatoren wie Peroxodisulfat oder mit Hilfe von Redoxinitiatoren, erzeugt werden.

Bevorzugt sind radikalisch hergestellte Emulsionspfropfpolymerisate auf teilchenförmige, hochvernetzte Kautschuke (Dien- oder Alkylacrylatkautschuke) mit Gelgehalten $> 80$ Gew.-% und mittleren Teilchendurchmessern (d50) von 80 bis 800 nm.

Besonders geeignet sind technisch gebräuchliche ABS-Polymerisate.

Mischungen von Vinyl-Homopolymerisaten und/oder Vinyl-Copolymerisaten mit Pfropfpolymerisaten sind ebenfalls geeignet.

Bevorzugte andere Thermoplasten b) sind auch thermoplastische Polyurethane. Dies sind Reaktionsprodukte aus Diisocyanaten, ganz oder überwiegend aliphatischen Oligo-und/oder Polyestern und/oder -ethern sowie einem oder mehreren Kettenverlängerern. Diese thermoplastischen Polyurethane sind im wesentlichen linear und besitzen thermoplastische Verarbeitungscharakteristiken.

Die thermoplastischen Polyurethane sind bekannt oder können nach bekannten Verfahren (siehe beispielsweise US-PS 3214411, J.H. Saunders und K.C. Frisch, "Polyurethanes, Chemistry and Technology", Vol II, Seiten 299 bis 451, Interscience Publishers, New York, 1964 und Mobay Chemical Coporation, "A Processing Handbook for Texin Urethane Elastoplastic Materials", Pittsburgh, PA) hergestellt werden.

Ausgangsmaterialien zur Herstellung der Oligoester und Polyester sind beispielsweise Adipinsäure, Bernsteinsäure, Subecinsäure, Sebacinsure, Oxalsäure, Methyladipinsäure, Glutarsäure, Pimelinsäure, Azelainsäure, Phthalsäure, Terephthalsäure und Isophthalsäure.

Adipinsäure ist hierbei bevorzugt.

Als Glykole zur Herstellung der Oligoester und Polyester kommen beispielsweise Ethylenglykol, 1,2- und 1,3-Propylenglykol, 1,2-, 1,3-, 1,4-, 2,3-, 2,4-Butandiol, Hexandiol, Bishydroxymethylcyclohexan, Diethylenglykol und 2,2-Diemthyl-propylenglykol in Betracht. Darüber hinaus können gemeinsam mit den Glykolen kleine Mengen, bis zu 1 Mol-%, tri- oder höher funktionelle Alkohole, z.B. Trimethylolpropan, Glycerin, Hexantriol usw. eingesetzt werden.

Die resultierenden Hydroxyl-oligo-oder -polyester haben ein Molekulargewicht von wenigstens 600, eine Hydroxylzahl von ca. 25 bis 190, vorzugsweise ca. 40 bis 150, eine Säurezahl von ca. 0,5 bis 2 und einen Wassergehalt vo ca. 0,01 bis 0,2%.

Oligoester bzw. Polyester sind auch oligomere oder polymere Lactone, wie beispielsweise Oligo-caprolacton oder Poly-caprolacton, und aliphatische Polycarbonate, wie beispielsweise Poly-butandiol-(1,4)-carbonat oder Poly-hexandiol-(1,6)-carbonat.

Ein besonders geeigneter Oligorest, der als Ausgangsmaterial für die thermoplastischen Polyurethane verwendet werden kann, wird aus Adipinsäure und einem Glykol hergestellt, das wenigstens eine primäre Hydroxylgruppe besitzt. Die Kondensation wird beendet, wenn eine Säurezahl von 10, vorzugsweise ca. 0,5 bis 2 erreicht ist. Das während der Reaktion entstehende Wasser wird damit gleichzeitig oder hinterher abgetrennt, so daß der Wassergehalt am Ende im Bereich von ungefähr 0,01 bis 0,05%, vorzugsweise 0,01 bis 0,02 liegt.

Oligo- bzw. Polyether zur Herstellung der thermoplastischen Polyurethane sind beispielsweise solche auf Basis von Tetramethylenglykol, Propylenglykol und Ethylenglkyol.

Polyacetale sind ebenfalls als Polyether zu verstehen und einsetzbar.

Die Oligoether bzw. Polyether sollen mittlere Molekulargewichte $\overline{M}n$ (Zahlenmittel, ermittelt über die OH-Zahl der Produkte) von 600 bis 2000 vorzugsweise von 1000 bis 2000 haben.

Als organisches Diisocyanat wird zur Herstellung der Polyurethane vorzugsweise 4,4′-Diphenylmethandiisocyanat verwendet. Es sollte weniger als 5% 2,4′-Diphenylmethan-diisocyanat und weniger als 2% des Dimeren von Diphenylmethan-diisocyanat enthalten. Es ist weiterhin wünschenswert, daß die Acidität, gerechnet als HCl im Bereich von c. 0,005 bis 0,2% liegt. Die Acidität, gerechnet als % HCl, wird durch Extraktion des Chlorids aus dem Isocyanat in heißer, wäßriger Methanol-Lösung oder durch Freisetzung des Chlorides bei Hydrolyse mit Wasser und Titration des Extraktes mit Standard-Silbernitrat-Lösung bestimmt, um die darin vorhandene Chlorid-Ionen-Konzentration zu erhalten.

Es können auch andere Diisocyanate zur Herstellung der thermoplastischen Polyurethane verwendet werden, beispielsweise die Diisocyante des Ethylens, Ethylidens, Propylens, Butylens, Cyclopentylens-1,3, Cyclohexylens-1,4, Cyclohexylens-1,2, des 2,4-Tolylens, des 2,6-Tolylens, des p-Phenylens, des n-Phenylens, des Xylens, des 1,4-Naphthylens, des 1,5-Naphthylens, des 4,4′-Diphenylens, das 2,2-Diphenylpropan-4,4′-diisocyanat, das Azobenzol-4,4′-diisocyanat, des Diphenylsulfon-4,4′-diisocyanat, das Dichlorhexanmethylendiisocyanat, das Pentamethylen-diisocyanat, das Hexamethylen-diisocyanat, das 1-Chlorbenzol-2,4-diisocyanat, das Furfuryl-diisocyanat, das Dicyclohexylmethan-diisocyanat, das Isophorondiisocyanat, das Diphenylethan-diisocyanat und Bis(-isocyanatophenyl)-ether von Ethylenglykol, Butandiol etc.

Als Kettenverlängerer können organische difunktionelle Verbindungen verwendet werden, die aktiven, mit Isocyanaten reaktiven, Wasserstoff enthalten, z.B. Diole, Hydroxycarbonsäuren, Dicarbonsäuren, Diamine und Alkanolamine und Wasser. Als solche sind beispielsweise Ethylen-, Propylen-, Butylenglykol, 1,4-Butandiol, Butandiol, Butindiol, Xylylenglykol, Amylenglykol, 1,4-Phenylen-bis-β-hydroxy-ethylether, 1,3-Phenylen-bis-β-hydroxyethylether, Bis-(hydroxymethyl-cyclohexan), Hexandiol, Adipinsäure, ω-Hydroxycapronsäure, Thiodiglykol, Ethylendiamin-, Propylen, Butylen-, Hexamethylen-, Cyclohexylen-, Phenylen-, Toluylen-, Xylylendiamin, Diaminodicyclohexylmethan, Isophorondiamin, 3,3′-Dichlorbenzidin, 3,3′-Dinitrobenzidin, Ethanolamin, Aminopropylalkohol, 2,2-Dimethyl-propanolamin, 3-Aminocyclohexylalkohol und p-Amiobenzylalkohol zu nennen. Das Molverhältnis Oligo- bzw. Polyester zu bifunktionellen Kettenverlängerer bewegt sich im Bereich 1 : 1 bis 1 : 50, vorzugsweise 1 : 2 bis 1 : 30.

Außer difunktionellen Kettenverlängerern können auch in untergeordneten Mengen bis zu etwa 5 Mol-%, bezogen auf Mole eingesetzten bifunktionellen Kettenverlängerer, trifunktionelle oder mehr als trifunktionelle Kettenverlängerer eingesetzt werden.

Derartige trifunktionelle oder mehr als trifunktionelle Kettenverlängerer sind beispielsweise Glycerin, Trimethylolpropan, Hexantriol, Pentaerythrit und Triethanolamin.

Monofunktionelle Komponenten, beispielsweise Butanol, können auch zur Herstellung der thermoplastischen Polyurethane eingesetzt werden.

Die als Bausteine für die thermoplastischen Polyurethane genannten Diisocyanate, Oligoester, Polyester, Polyether. Kettenverlängerer und monofunktionellen Komponenten sind entweder literaturbekannt oder nach literaturbekannten Verfahren erhältlich.

Die bekannte Herstellung der Polyurethane kann beispielsweise wie folgt durchgeführt werden :

So können beispielsweise die Oligo- bzw. Polyester, die organischen Diisocyanate und die Kettenverlängerer für sich vorzugsweise auf eine Temperatur von ca. 50 bis 220°C erhitzt und dann vermischt werden. Vorzugsweise werden die Oligo- bzw. Polyester zunächst einzeln erhitzt, dann mit den Kettenverlängerern gemischt und die erhaltene Mischung mit dem vorerhitzten Isocyanat vermischt.

Das Mischen der Ausgangskomponenten zur Herstellung der Polyurethane kann mit irgendeinem mechanischen Rührer erfolgen, der intensive Mischung innerhalb kurzer Zeit erlaubt. Falls die Viskosität der Mischung während des Rührens vorzeitig zu schnell steigen sollte, kann entweder die Temperatur gesenkt oder eine kleine Menge (0,001 bis 0,05 Gew.-%, bezogen auf Ester) Zitronensäure oder ähnliches zugegeben werden, um die Geschwindigkeit der Reaktion zu verringern. Zur Erhöhung der Reaktionsgeschwindigkeit können geeignete Katalysatoren, wie z.B. tertiäre Amine, die in dem US-Patent 2729618 genannt werden zur Anwendung kommen.

Bevorzugte andere Thermoplasten sind auch sogenannte "LC-Polymere". Als LC-Polymere werden Polymere bezeichnet, die flüssigkristalline Schmelzen bilden können. Derartige Polymere, die auch als "thermotrop" bezeichnet werden, sind hinreichend bekannt (siehe beispielsweise EP-OS 0131846, EP-OS 0132637 und EP-OS 0134959). In den genannten Literaturstellen ist weitere Literatur angezogen und darüber hinaus die Ermittlung des flüssigkristallinen Zustandes von Polymerschmelzen beschrieben.

"LC-Polymere" sind beispielsweise aromatische Polyester auf Basis von gegebenenfalls substituierter p-Hydroxybenzoesäure, gegebenenfalls substituierten Iso- und/oder Terephthalsäuren, 2,7-Dihydroxynaphthalin und anderen Diphenolen (EP-OS 0131846), aromatische Polyester auf Basis von gegebenenfalls substituierter p-Hydroxybenzoesäure, Diphenolen, Kohlensäure und gegebenenfalls aromatischen Dicarbonsäuren (EP-OS 0132637) und aromatischer Polyester auf Basis von gegebenenfalls substituierter p-Hydroxybenzoesäure, 3-Chlor-4-hydroxybenzoesäure, Isophthalsäure, Hydrochinon und 3,4′- und/oder 4,4′-Dihydroxydiphenyl, 3,4′-

und/oder 4,4′-Dihydroxydiphenylether und/oder 3,4′- und/oder 4,4′-Dihydroxydiphenylsulfid (EP-OS 0134949).

Die LC-Polymere haben eine Persistenzlänge bei Zimmertemperatur zwischen 18 und 1300 Å, bevorzugt zwischen 25 und 300 Å, insbesondere zwischen 25 und 150 Å.

Die Persistenzlänge eines Polymeren bei Zimmertemperatur charakterisiert die mittlere Verknäuelung einer Molekülkette in einer verdünnten Lösung unter Theta-Bedingungen (vgl. z.B. P.J. Flory, "Principles of Polymer Chemistry", Cornell Univ. Press, Ithaca, New York) und die Hälfte der Kuhnschen Schrittlänge. Die Persistenzlänge kann mit verschiedenen Methoden in verdünnten Lösungen bestimmt werden, z.B. durch Lichtstreuung und Röntgenkleinwinkel-Messungen. Man kann nach geeigneter Präparation die Persistenzlänge auch mit Hilfe der Neutronenkleinwinkelstreuung im Festkörper bestimmen. Weitere theoretische und experimentelle Methoden sind z.B. in J.H. Wendorff in "Liquid Crystalline Order in Polymers", e.g. A. Blumstein, Academic Press 1978, S. f16 ff sowie in den in "S.M. Aharoni, Macromolecules 19, (1986), S. 429 ff" angegebenen Referenzen beschrieben.

Bevorzugte andere Thermoplasten sind auch aromatische Polyestercarbonate.

Erfindungsgemäß als Thermoplast b) einsetzbare aromatische Polyester und Polyestercarbonate sind aus mindestens einem aromatischen Bisphenol, z.B. der Formel (VII), aus mindestens einer aromatischen Dicarbonsäure und gegebenenfalls aus Kohlensäure aufgebaut. Geeignete aromatische Dicarbonsäuren sind beispielsweise Orthophthalsäure, Terephthalsäure, Isophthalsäure, tert.-Butylisophthalsäure, 3,3′-Diphenyldicarbonsäure, 4,4′-Diphenyldicarbonsäure, 4,4′-Benzophenondicarbonsäure, 3,4′-Benzophenondicarbonsäure, 4,4′-Diphenyletherdicarbonsäure, 4,4′-Diphenylsulfondicarbonsäure, 2,2-Bis-(4-carboxyphenyl)-propan, Trimethyl-3-phenylindan-4,5′-dicarbonsäure.

Von den aromatischen Dicarbonsäuren werden besonders bevorzugt die Terephthalsäure und/oder Isophthalsäure eingesetzt.

Aromatische Polyester und Polyestercarbonate können nach Verfahren hergestellt werden, wie sie für die Polyester- bzw. Polyestercarbonat-Herstellung aus der Literatur bekannt sind, so z.B. nach Verfahren in homogener Lösung, nach Schmelzumesterungsverfahren und nach dem Zweiphasengrenzflächenverfahren. Bevorzugt werden Schmelzumesterungsverfahren und insbesondere das Zweiphasengrenzflächenverfahren angewandt.

Schmelzumesterungsverfahren (Acetatverfahren und Phenylesterverfahren) werden beispielsweise in den US-PS 3494885, 4386186, 4661580, 4680371 und 4680372, den EP-A 26120, 26121, 26684, 28030, 39845, 91602, 97970, 79075, 146887, 156103, 234913, 234919 und 240301 sowie den DE-A 1495626, 2232977 beschrieben. Das Zweiphasengrenzflächenverfahren wird beispielsweise beschrieben in den EP-A 68014, 88322, 134898, 151750, 182189, 219708, 272426, in DE-OS 2940024, 3007934, 3440020 und in Polymer Reviews, Volume 10, "Condensation Polymers by Interfacial and Solution Methods", Paul W. Morgan, Interscience Publishers, New York 1965, Kap. VIII, S. 325, Polyester.

Beim Acetatverfahren werden im allgemeinen Bisphenoldiacetat bzw. beim Phenylesterverfahren werden im allgemeinen Bisphenol, aromatische Dicarbonsäure oder Diphenylester der aromatischen Dicarbonsäure und gegebenenfalls Diphenylcarbonat unter Phenolabspaltung und gegebenenfalls $CO_2$-Abspaltung zum Polyester bzw. Polyestercarbonat umgesetzt. Beim Zweiphasengrenzflächenververfahren dienen als Ausgangsstoffe zur Herstellung von Polyestern und Polyestercarbonaten, im allgemeinen Alkalibisphenolat, aromatisches Dicarbonsäuredichlorid und gegebenenfalls Phosgen. Bei dieser Kondensationsreaktion werden der Polyester bzw. das Polyestercarbonat unter Alkalichloridbildung hergestellt. Im allgemeinen ist das gebildete Salz in der wäßrigen Phase gelöst, währende der gebildete Polyester bzw. das gebildete Polyestercarbonat in der organischen Phase gelöst vorliegt und daraus isoliert wird.

Bevorzugte Elastomere b3) für die Komponente b) zur Herstellung der erfindungsgemäßen Mischungen sind die vorstehend erwähnten Polyurethane, soweit sie elastischer Natur sind, Styrol, Butadien-Blockcopolymere, die teilweise hydriert sein können (beispielsweise Kraton G® der Shell), die vorstehend für die Pfropfpolymerisate erwähnten Kautschuke, die Pfropfpolymerisate selbst, soweit sie elastisch sind sowie elastische Polycarbonat-Polyether-Blockcopolymere.

Diese Elastomeren sind bekannt.

Die Folien, beziehungsweise Verbundfolien können flach, hohl, kugelförmig, schlauchförmig und hohlfaserförmig sein. Derartige Folien sind nach bekannten Verfahren durch Verformen, Tiefziehen, Blasen etc. erhältlich.

Die erfindungsgemäßen Folien, insbesondere die Verbundfolien, finden Verwendung beispielsweise für koch- und ofenfeste dichte Verpackung und für mikrowellenfeste Verpackungen, je nachdem, mit welcher Komponente b) die erfindungsgemäße Verbundfolie aufgebaut ist.

Die erfindungsgemäßen Verbundfolien können durch Coextrusion der thermoplastischen Kunststoffe mit den erfindungsgemäßen Polycarbonaten in einem Arbeitsgang erzeugt werden.

Die Folien aus den erfindungsgemäßen Polycarbonaten und die erfindungsgemäßen Verbundfolien auf

Basis dieser Folien aus den erfindungsgemäßen Polycarbonaten können als homogene Membranen, Kompositionsmembranen oder asymmetrische Membranen verwendet werden.

In den nachfolgenden Beispielen wird die relative Viskosität gemessen an 0,5 Gew.-%igen Lösungen des Polycarbonats in $CH_2Cl_2$.

Die Einfriertemperatur oder Glastemperatur wird gemessen durch Differential Scanning Calorimetry (DSC).

Beispiel B.1

31,0 g (0,1 Mol) des Diphenols gemäß Beispiel (A.1), 33,6 g (0,6 Mol) KOH und 560 g Wasser werden in einer Inertgas-Atmosphäre unter Rühren gelöst. Dann fügt man eine Lösung von 0,188 g Phenol in 560 ml Methylenchlorid zu. In die gut gerührte Lösung wurden bei pH 13 bis 14 und 21 bis 25°C 19,8 g (0,2 Mol) Phosgen eingeleitet. Danach wird 0,1 ml Ethylpyridin zugegeben und noch 45 Minuten gerührt. Die bisphenolatfreie wäßrige Phase wird abgetrennt, die organische Phase nach Ansäuern mit Phosphorsäure mit Wasser neutral gewaschen und vom Lösungsmittel befreit. Das Polycarbonat zeigte eine relative Lösungsviskosität von 1,259.

Die Glastemperatur des Polymers wurde zu 233°C bestimmt (DSC).

Beispiel B.2

68,4 g (0,3 Mol) Bisphenol A (2,2-Bis-(4-hydroxyphenyl)-propan, 217,0 g (0,7 Mol) Diphenol gemäß Beispiel (A.1), 336,6 g (6 Mol KOH und 2700 g Wasser werden in einer Inertgas-Atmosphäre unter Rühren gelöst. Dann fügt man eine Lösung von 1,88 g Phenol in 2500 ml Methylenchlorid zu. In die gut gerührte Lösung wurden bei pH 13 bis 14 und 21 bis 25°C 198 g (2 Mol) Phosgen eingeleitet. Danach wird 1 ml Ethylpiperidin zugegeben und noch 45 Min. gerührt. Die bisphenolatfreie wäßrige Phase wird abgetrennt, die organische Phase nach Ansäuern mit Phosphorsäure mit Wasser neutral gewaschen und vom Lösungsmittel befreit. Das Polycarbonat zeigte eine relative Lösungsviskosität von 1,336.

Die Glastemperatur des Polymers wurde zu 212°C bestimmt (DSC).

Beispiel B.3

Wie in Beispiel B.2 wurde eine Mischung aus 114 g (0,5 Mol) Bisphenol A und 155 g (0,5 Mol) Diphenol gemäß Beispiel (A.1) zum Polycarbonat umgesetzt.

Das Polycarbonat zeigte eine relative Lösungsviskosität von 1,386.

Die Glastemperatur des Polymer wurde zu 195°C bestimmt (DSC).

Beispiel B.4

Wie in Beispiel B.2 wurde eine Mischung aus 159,6 g (0,7 Mol) Bisphenol A und 93 g (0,3 Mol) Diphenol gemäß Beispiel (A.3) zum Polycarbonat umgesetzt.

Das Polycarbonat zeigte eine relative Lösungsviskosität von 1,437.

Die Glastemperatur des Polymers wurde zu 180°C bestimmt (DSC).

Beispiel B.5

31,0 g (0,1 Mol) Diphenol gemäß Beispiel (A.3), 24,0 g (0,6 Mol) NaOH und 270 g Wasser werden in einer Inertgas-Atmosphäre unter Rühren gelöst. Dann fügt man eine Lösung von 0,309 g 4-(1,1,3,3-Tetramethylbutyl)-phenol in 250 ml Methylenchlorid zu. In die gut gerührte Lösung wurden bei pH 13 bis 14 und 21 bis 25°C 19,8 g (0,2 Mol) Phosgen eingeleitet. Danach wird 0,1 ml Ethylpiperidin zugegeben und noch 45 Min. gerührt. Die bisphenolatfreie wäßrige Phase wird abgetrennt, die oganische Phase nach Ansäuern mit Phosphorsäure mit Wasser neutral gewaschen und vom Lösungsmittel befreit. Das Polycarbonat zeigte eine relative Lösungsviskosität von 1,314.

Die Glastemperatur des Polymers wurde zu 234°C bestimmt (DSC).

Zur Abschätzung des UV-Beständigkeit der neuen Polycarbonate wurde die Primärradikalbildung bei UV-Bestrahlung mit einer Quecksilberdampflampe (Kantenfilter 305 nm) im Vergleich zu einem Polycarbonat auf Basis des 2,2-Bis-(4-hydroxyphenyl)-propans bestimmt. Es zeigte sich, daß das Polycarbonat gemäß Beispiel B1 eine geringere Primärradikalbildungsrate und daher eine höhere UV-Beständigkeit aufweist.

Beispiel B.6

148,2 g (0,65 Mol) 2,2-Bis-(4-hydroxyphenyl)-propan, 108,5 g (0,35 Mol) Diphenol gemäß Beispiel (A.1), 336,6 g (6 Mol) KOH und 2700 g Wasser werden in einer Inertgas-Atmosphäre unter Rühren gelöst. Dann fügt man eine Lösung von 8,86 g 4-(1,1,3,3-Tetramethylbutyl-phenol in 2500 ml Methylenchlorid zu. In die gut gerührte Lösung wurden bei pH 13-14 und 21-25°C 198 g (2 Mol) Phosgen eingeleitet. Danach wird 1 ml Ethylpiperidin zugegeben und noch 45 Minuten gerührt. Die bisphenolatfreie wäßrige Phase wird abgetrennt, die organische Phase nach Ansäuern mit Phosphorsäure mit Wasser neutral gewaschen und vom Lösungsmittel befreit. Das Polycarbonat zeigte eine rel. Lösungsviskosität von 1,20.

Beispiel B.7

3,875 kg (12,5 Mol) Bisphenol gemäß Beispiel (A.2) werden in 6,675 kg 45%iger NaOH und 30 l Wasser in einer Inertgasatmosphäre unter Rühren gelöst. Dann werden 9,43 l Methylenchlorid, 11,3 l Chlorbenzol und 23,5 g Phenol zugegeben. In die gut gerührte Lösung werden bei pH 13-14 und 20-25°C 2,475 kg Phosgen eingeleitet. Nach Beendigung des Einleitens werden 12,5 ml N-Ethylpiperidin zugegeben. Man läßt 45 min. nachreagieren. Die bisphenolatfreie wäßrige Phase wird abgetrennt, die organische Phase mit Phosphorsäure angesäuert und anschließend elektrolytfrei gewaschen und vom Lösungsmittel befreit.

rel. Viskosität : 1,300
Glastemperatur : 238°C

Beispiel B.8

15,5 g (0,05 Mol) Bisphenol gemäß Beispiel (A.3), 13,4 g (0,05 Mol) Bis- (4-hydroxyphenyl)-cyclohexan (Bisphenol Z), 24,0 g (0,6 Mol) NaOH werden in 362 ml Wasser in einer Inertgasatmosphäre unter Rühren gelöst. Dann werden 0,516 g 4-(1,1,3,3-Tetramethylbutyl)phenol gelöst in 271 ml Methylenchlorid zugegeben. In die gut gerührte Lösung werden bei pH 13-14 und 20-25°C 19,8 g Phosgen eingeleitet. 5 Minuten nach Beendigung des Einleitens werden 0,1 ml N-Ethylpiperidin zugegeben. Man läßt 45 Minuten nachreagieren. Die bisphenolatfreie wäßrige Phase wird abgetrennt, die organische Phase mit Phosphorsäure angesäuert und anschließend neutral gewaschen und vom Lösungsmittel befreit.

rel. Viskosität : 1,297
Glastemperatur : 208°C

Beispiel B.9

15,5 g (0,05 Mol) Bisphenol gemäß Beispiel (A.1), 17,6 g (0,05 Mol) 4,4′-Dihydroxytetraphenylmethan, 24,0 g (0,6 Mol) NaOH werden in 411 ml Wasser in einer Inertgasatmosphäre unter Rühren gelöst. Dann werden 0,516 g 4-(1,1,3,3-Tetramethylbutyl)phenol gelöst in 308 ml Methylenchlorid zugegeben. In die gut gerührte Lösung werden bei pH 13-14 und 20-25°C 19,8 g Phosgen eingeleitet. 5 Minuten nach Beendigung des Einleitens werden 0,1 ml N-Ethylpiperidin zugegeben. Man läßt 45 min. nachreagieren. Die bisphenolatfreie wäßrige Phase wird abgetrennt, die organische Phase mit Phosphorsäure angesäuert und anschließend neutral gewaschen und vom Lösungsmittel befreit.

rel. Viskosität : 1,218
Glastemperatur : 212°C

Beispiel B.10

18,3 g (0,05 Mol) Bisphenol gemäß Beispiel (A.4), 23,6 g (0,42 Mol) KOH werden in 100 ml Wasser in einer Inertgasatmosphäre unter Rühren gelöst. Dann werden 100 ml Methylenchlorid zugegeben. In die gut gerührte Lösung werden bei pH 13-14 und 20-25° 17,3 g Phosgen eingeleitet. 5 Minuten nach Beendigung des Einleitens werden 0,3 ml N-Ethylpiperidin zugegeben. Man läßt 45 min. nachreagieren. Die bisphenolatfreie wäßrige Phase wird abgetrennt, die organische Phase mit Phosphorsäure angesäurert und anschließend neutral gewaschen und vom Lösungsmittel befreit.

rel. Viskosität : 1,310

Glastemperatur : 241°C

Beispiel B.11

29,6 g (0,1 Mol) Bisphenol gemäß Beispiel (A.5), 24,0 g (0,6 Mol) NaOH werden in 370 ml Wasser in einer Inertgasatmosphäre unter Rühren gelöst. Dann werden 0,413 g 4-(1,1,3,3-Tetramethylbutyl)phenol gelöst in 277 ml Methylenchlorid zugegeben. In die gut gerührte Lösung werden bei pH 13-14 und 20-25°C 19,8 g Phosgen eingeleitet. 5 Minuten nach Beendigung des Einleitens werden 0,1 ml N- Ethylpiperidin zugegeben. Man läßt 45 min. nachreagieren. Die bisphenolatfreie wäßrige Phase wird abgetrennt, die organische Phase mit Phosphorsäure angesäuert und anschließend neutral gewaschen und vom Lösungsmittel befreit.

rel. Viskosität : 1,370
Glastemperatur : 193°C

Beispiel B.12

62,0 g (0,2 Mol) Bisphenol gemäß Beispiel (A.1), 182,4 g (0,8 Mol) Bisphenol A, 240 g (6 Mol) NaOH werden in 2400 ml Wasser in einer Inertgasatmosphäre unter Rühren gelöst. Dann werden 6,603 g 4-(1,1,3,3-Tetramethylbutyl)phenol gelöst in 2400 ml Methylenchlorid zugegeben. In die gut gerührte Lösung werden bei pH 13-14 und 20-25°C 198 g Phosgen eingeleitet. 5 Minuten nach Beendigung des Einleitens wird 1 ml N-Ethylpiperidin zugegeben. Man läßt 45 min. nachreagieren. Die bisphenolatfreie wäßrige Phase wird abgetrennt, die organische Phase mit Phosphorsäure angesäuert und anschließend neutral gewaschen und vom Lösungsmittel befreit.

rel. Viskosität : 1,298
Glastemperatur : 172°C

Beispiel B.13

170,5 g (0,55 Mol) Bisphenol gemäß Beispiel (A.3), 102,6 g (0,45 Mol) Bisphenol A, 240 g (6 Mol) NaOH werden in 2400 ml Wasser in einer Inertgasatmosphäre unter Rühren gelöst. Dann werden 5,158 g 4-(1,1,3,3-Tetramethylbutyl)-phenol gelöst in 2400 ml Methylenchlorid zugegeben. In die gut gerührte Lösung werden bei pH 13-14 und 20-25°C 198 g Phosgen eingeleitet. 5 Minuten nach Beendigung des Einleitens wird 1 ml N-Ethylpiperidin zugegeben. Man läßt 45 min. nachreagieren. Die bisphenolatfreie wäßrige Phase wird abgetrennt, die organische Phase mit Phosphorsäure angesäuert und anschließend neutral gewaschen und vom Lösungsmittel befreit.

rel. Viskosität : 1,302
Glastemperatur : 203°C

Beispiel B.14

108,5 g (0,35 Mol) Bisphenol gemäß Beispiel (A.1), 148.2 g (0,65 Mol) Bisphenol A, 240 g (6 Mol) NaOH werden in 2400 ml Wasser in einer Inertgasatmosphäre unter Rühren gelöst. Dann werden 6,189 g 4-(1,1,3,3-tetramethylbutyl)phenol gelöst in 2400 ml Methylenchlorid zugegeben. In die gut gerührte Lösung werden bei pH 13-14 und 20-25°C 198 g Phosgen eingeleitet. 5 Minuten nach Beendigung des Einleitens wird 1 ml N-Ethylpiperidin zugegeben. Man läßt 45 min. nachreagieren. Die bisphenolatfreie wäßrige Phase wird abgetrennt, die organische Phase mit Phosphorsäure angesäuert und anschließend neutral gewaschen und vom Lösungsmittel befreit.

rel. Viskosität : 1,305
Glastemperatur : 185°C

Beispiel C

Aus dem Copolycarbonat gemäß Beispiel (B.6) und aus einem Bisphenol-A- Homopolycarbonat mit $\eta_{rel}$ = 1,20 wurden auf einer Netstal-Spritzgußmaschine Compact Discs mit einem Durchmesser von 12 cm hergestellt (Massetemperatur 330 bis 350°C). Es wurde die Doppelbrechung in axialer Richtung anhand von Gangunterschiedsmessungen durch Verwendung eines üblichen Komparators mittels eines Polarisationsmikroskops überprüft. Die Transparenz wurde visuell beurteilt, die Glastemperatur durch DSC bestimmt.

| Material | Gangunterschied [nm/mm] | $T_g$ [°C] | Transparenz |
|---|---|---|---|
| Beispiel B6 | + 13 | 185 | ja |
| Bisphenol-A-Polycarbonat | + 12 | 145 | ja |

Beispiel D (Folienherstellung)

D.1

20 g des Polycarbonats aus Beispiel B.1 wurden in 200 ml Methylenchlorid unter ständigem Rühren bei 30°C gelöst, die Lösung eingedickt und anschließend durch Ausgießen auf einer ebenen Glasplatte bei 25°C eine Folie einer Dicke von 204 µm hergestellt. Diese Folie wurde 4 Stunden bei 90°C unter Vakuum getrocknet, anschließend wurde die Gasdurchlässigkeit durch diese Folie gemessen.

Bestimmung der Gasdurchlässigkeit (Permeation) von Polymermembranen

Der Durchgang eines Gases durch eine dichte Polymermembran wird durch einen Lösungs-Diffusionsvorgang beschrieben. Die kennzeichnende Konstante für diesen Prozeß ist der Permeationskoeffizient P, der angibt, welches Gasvolumen V bei gegebener Druckdifferenz Δp in einer bestimmten Zeit t durch eine Folie bekannter Fläche F und Dicke d hindurchtritt. Für den stationären Zustand läßt sich aus den Differentialgleichungen des Permeationsvorgangs ableiten :

$$P = \frac{V \cdot d}{F \cdot t \cdot \Delta p} \qquad (1)$$

Darüber hinaus ist die Permeation abhängig von der Temperatur und dem Wassergehalt des Gases.

Die Meßanordnung besteht aus einem thermostatisierten Zwei-Kammer-System. Die eine Kammer ist für die Aufnahme des Vorgabegases und die andere für die Aufnahme des Permeates ausgelegt. Die Kammern sind durch die zu messende Polymermembran getrennt.

Beide Kammern werden auf $10^{-3}$ mbar evakuiert und die Vorgabekammer dann mit Gas gefüllt. Das permeierte Gas (inerte Gase) bewirkt dann in der Permeatkammer bei konstantem Volumen einen Druckanstieg, der mit einem Druckaufnehmer (Baratron der Fa. MKS) in Abhängigkeit von der Zeit bis in den stationären Gasdurchgang registriert wird. Aus dem Druckanstieg kann V (für Normaldruck und Normaltemperatur) errechnet werden, t ist bekannt. Δp wird unter Berücksichtigung des äußerdem Luftdruckes jeweils auf $10^5$ Pa eingestellt. Die Membranfläche F ist bekannt. Die Membrandicke d wird mittels Mikrometerschraube als Mittel aus 10 unabhängigen über die Membranfläche verteilten Dickenmessungen ermittelt.

Aus diesen Größen ist der Permeationskoeffizient P nach (1) zu bestimmen in der Dimension

$$\left[ \frac{cm^3 \ (NTP) \cdot mm}{m^2 \cdot 24h \ 10^5 \ Pa} \right]$$

wobei auf eine Membrandicke von 1 mm bezogen ist.
Weitere Meßparameter sind :

| | |
|---|---|
| Temperatur : | 25 ± 1°C |
| rel. Gasfeuchte : | 0% |

Ergebnis : Permeationskoeffizient

| | |
|---|---|
| für $O_2$ : | 280,8 |
| für $N_2$ : | 84,5 |
| für $CO_2$ : | 2174,0 |
| für $CH_4$ : | 149,4 |

Die Folie war noch bei 180°C formstabil.

D.2 (Vergleichsbeispiel)

Entsprechend Beispiel D.1 wurde eine Folie aus Bisphenol-A-Polycarbonat mit einer relativen Viskosität von 1,28 hergestellt (Dicke : 154 µm) und vermessen.
Ergebnis : Permeationskoeffizient

| | |
|---|---|
| für $O_2$ : | 72,0 |
| für $N_2$ : | 366,0 |
| für $CO_2$ : | 35,0 |
| für $CH_4$ : | 27,0 |

Bei 180°C war diese Folie nicht mehr formstabil.

Beispiel D.3

Wie in Beispiel D.1 beschrieben, wird aus 20 g des Polycarbonates aus Beispiel B.12 eine Folie der Dicke 92 µm hergestellt und anschließend die Gasdurchlässigkeit gemessen.

Beispiel D.4

Wie in Beispiel D.1 beschrieben, wird aus 20 g des Polycarbonates aus Beispiel B.13 eine Folie der Dicke 95 µm hergestellt und anschließend die Gasdurchlässigkeit gemessen.

Beispiel D.5

Wie in Beispiel D.1 beschrieben, wird aus 20 g des Polycarbonates aus Beispiel B.14 eine Folie der Dicke 89,7 µm hergestellt und anschließend die Gasdurchlässigkeit gemessen.

Beispiel D.6

Das Polycarbonat aus Beispiel B.7 wird in einem Extruder aufgeschmolzen (Temperatur : 360-370°C) und über Breitschlitzdüsen zu einer Folie mit einer Dicke von 163 µm extrudiert und anschließend die Gasdurchlässigkeit gemessen.

Beispiel D.7

31 g (0,1 Mol) Bisphenol A.1, 24 g (0,6 Mol) NaOH werden in 270 ml Wasser in einer Inertgasatmosphäre unter Rühren gelöst. Dann werden 250 ml Methylenchlorid zugegeben. In die gut gerührte Lösung werden bei pH 13-14 und 20-25°C 19,8 g Phosgen eingeleitet. 5 Minuten nach Beendigung des Einleitens wird 0,1 ml N-Ethylpiperidin zugegeben. Man läßt 45 min nachreagieren. Die bisphenolatfreie wäßrige Phase wird abgetrennt, die organische Phase mit Phosphorsäure angesäuert und anschließend neutral gewaschen. Aus der aufkonzentrierten Lösung in Methylenchlorid wurde ein Film gegossen, der klar transparent war.

GPC-Analyse : Das Molekulargewicht wurde auf Basis einer Kalibrierung mit Bisphenol-A-Polycarbonat durchgeführt.
$M_w = 246000$, $M_n = 38760$

Ergebnisse Permeationswerte:

| | Permeationsgase | | | |
|---|---|---|---|---|
| Probe | $N_1$ | $O_2$ | $CO_2$ | $CH_4$ |
| D.3 | 23,9 | 109,2 | 634,9 | 30,2 |
| D.4 | 49,7 | 227,9 | 1629,5 | 64,3 |
| D.5 | 33,6 | 138,8 | 828,1 | 46,8 |
| D.6 | 78,2 | 400,5 | 2555,0 | n.b. |

n.b.: nicht bestimmt

D.8 (Verbundfolie)

Die nach D.1 und D.2 hergestellten Folien wurden nach Ausdampfen des Lösungsmittels aufeinander gelegt und bei 235°C und einem Druck von etwa 234 bar 4 Minuten lang zu einer Folie mit einer Dicke von etwa 307 µm verpreßt.
Die Gasdurchlässigkeit der Verbundfolie wurde wie in D.1 beschrieben, gemessen.
Ergebnis : Permeationskoeffizient

für $O_2$ : 208,3
für $CO_2$ : 1209,4
für $CH_4$ : 77,1

Diese Verbundfolie war auch noch bei 180°C formstabil.

Beispiel D.9

Verbundfolie aus Polycarbonat gemäß Beispiel B.14 und Polymethylmethacrylat

Eine Polymethylmethacrylat-Folie (PMMA) mit einer Dicke von 130 µm und eine Folie aus dem Polycarbonat B.14 der Dicke 131 µm werden bei 160°C nach 30 Sekunden Vorwärmen unter einem Preßdruck von 200 bar verpreßt. Die Gasdurchlässigkeiten der Verbundfolie wurde wie in Beispiel D.1 beschrieben gemessen.

Beispiel D.10

Verbundfolie aus Polycarbonat gemäß Beispiel B.14 und Polystyrol

Eine Polystyrol-Folie (Polystyrol N 168 der BASF AG) mit einer Dicke von 78 µm und eine Folie aus dem Polycarbonat B.14 der Dicke 101 µm werden bei 160°C nach 30 Sekunden Vorwärmen unter einem Preßdruck von 200 bar 30 Sekunden lang zu einer Verbundfolie der Dicke 168 µm verpreßt. Die Gasdurchlässigkeiten der Verbundfolie wurde wie in Beispiel D.1 beschrieben gemessen.

Ergebnisse Permeationswerte:

| | Permeationsgase | | | |
|---|---|---|---|---|
| Probe | $N_2$ | $O_2$ | $CO_2$ | $CH_4$ |
| D.9 | 0,7* | 4,5 | 20,3 | 0,42* |
| D.10 | 18,0 | 102,9 | 488,5 | 25,6 |

*: Da sich bei dieser Verbundfolie ein sehr geringer Druckanstieg nach Gasvorgabe in einer Meßzelle zeigte, wurde der Permeationswert aus dem Permeat nach 3 Tagen Permationszeit bestimmt.

## Patentansprüche

1. Dihydroxydiphenylcycloalkane der Formel

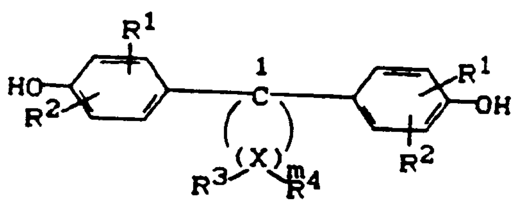

(I),

worin

$R^1$ und $R^2$ unabhängig voneinander Wasserstoff, Halogen, $C_1$-$C_8$-Alkyl, $C_5$-$C_6$-Cycloalkyl, $C_6$-$C_{10}$-Aryl und $C_7$-$C_{12}$-Aralkyl,

m, 4 oder 5

$R^3$ und $R^4$ für jedes X individuell wählbar, unabhängig voneinander Wasserstoff, $C_1$-$C_6$-Alkyl und

X Kohlenstoff bedeuten

mit der Maßgabe, daß an mindestens einem Atom X $R^3$ und $R^4$ gleichzeitig Alkyl bedeuten.

2. Dihydroxydiphenylcycloalkane der Formel (I) gemäß Anspruch 1, dadurch gekennzeichnet, daß in Formel (I) beide zu C-1 $\alpha$-ständigen X-Atome nicht dialkyl-substituiert sind.

3. Dihydroxydiphenylcycloalkane der Formel (I) gemäß Anspruch 2, dadurch gekennzeichnet, daß in Formel (I) ein dialkyl-substituiertes X-Atom in $\beta$-Stellung zu C-1 ist.

4. 1,1-Bis-(4-hydroxyphenyl)-3,3,5-trimethylcyclohexan.

5. Verfahren zur Herstellung der Dihydroxydiphenylcycloalkane der Formel (I)

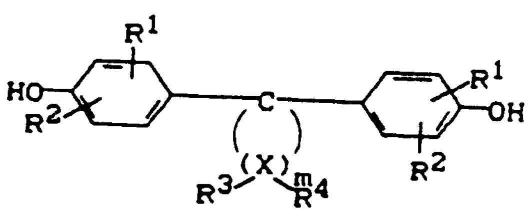

(I),

worin
X, $R^1$, $R^2$, $R^3$, $R^4$ und m die in Anspruch 1 genannte Bedeutung haben, dadurch gekennzeichnet, daß man Phenole der Formel (V)

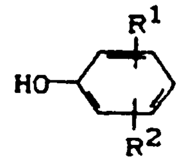

(V)

worin
$R^1$ und $R^2$ die für Formel (I) angegebene Bedeutung haben mit Ketonen der Formel (VI)

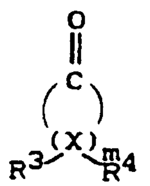

(VI)

worin
X, m, $R^3$ und $R^4$ die für Formel (I) angegebene Bedeutung haben, im Molverhältnis (V) : (VI) zwischen 2 : 1 und 10 : 1, vorzugsweise zwischen 2,5 : 1 und 6 : 1 bei Temperaturen zwischen – 30°C und 300°C, vorzugsweise zwischen – 15°C und 150°C und bei Drücken von 1 bis 20 bar, vorzugsweise von 1 bis 10 bar in Anwesenheit saurer Katalysatoren und gegebenenfalls in Anwesenheit von Co-Katalysatoen und/oder Lösungsmitteln und/oder wasserentziehenden Mitteln umsetzt.

6. Verwendung der Diphenole der Formel (I) des Anspruchs 1 zur Herstellung von hochmolekularen, thermoplastischen, aromatischen Polycarbonaten.

7. Verfahren zur Herstellung von hochmolekularen thermoplastischen, aromatischen Polycarbonaten aus Diphenolen, gegebenenfalls Kettenabbrechern und gegebenenfalls Verzweigern nach den bekannten Methoden der Polycarbonatherstellung, vorzugsweise nach dem Zweiphasengrenzflächenverfahren, dadurch gekennzeichnet, daß man als Diphenole solche der Formel (I) des Anspruchs 1 in Mengen von 100 Mol-% bis 2 Mol-%, bezogen jeweils auf die Gesamtmolmenge an eingesetzten Diphenolen, verwendet.

8. Hochmolekulare, thermoplastische, aromatische Polycarbonate mit $\overline{M}_w$ (Gewichtsmittelmolekulargewichten) von mindestens 10000 erhältlich nach dem Verfahren des Anspruchs 8.

9. Hochmolekulare, thermoplastische, aromatische Polycarbonate mit $\overline{M}_w$ (Gewichtsmittelmolekulargewichten) von mindestens 10000, die bifunktionelle Carbonatstruktureinheiten der Formel (Ia)

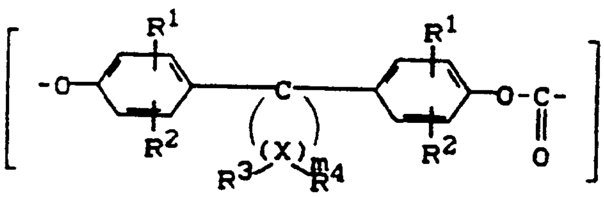

(Ia)

worin
X, $R^1$, $R^2$, $R^3$, $R^4$ und m die für die Formel (I) des Anspruchs 1 genannte Bedeutung haben, in Mengen von 100 Mol-% bis 2 Mol-%, bezogen jeweils auf die Gesamtmenge von 100 Mol-% an difunktionellen Carbonatstruktureinheiten im Polycarbonat enthalten.

10. Polycarbonate gemäß Anspruch 9, die die Struktureinheiten der Formel (Ia) in Mengen von 100 Mol-% bis 5 Mol-% enthalten.

11. Polycarbonate gemäß Anspruch 9, die zu 100 Mol-% komplementäre Mengen an difunktionellen Struktureinheiten der Formel

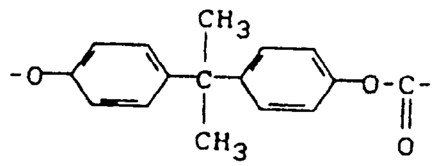

enthalten.

12. Polycarbonate gemäß 9, enthaltend Struktureinheiten der Formel (Ic)

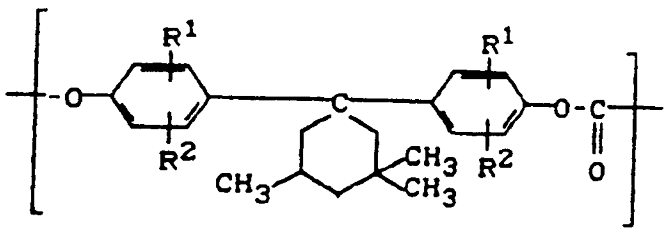 (Ic)

worin
$R^1$ und $R^2$ die für Formel (Ia) des Anspruchs 9, genannte Bedeutung haben.

13. Polycarbonate gemäß Anspruch 9, enthaltend Struktureinheiten der Formel (IIa)

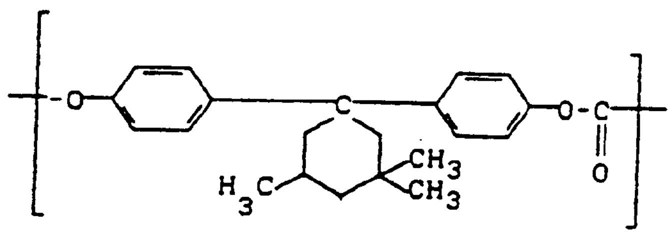 (IIa)

14. Folien aus den Polycarbonaten gemäß Anspruch 9.

15. Folien einer Dicke von 1-1500 µm aus den Polycarbonaten nach Anspruch 9.

16. Folien einer Dicke von 1-1500 µm aus den Polycarbonaten gemäß Anspruch 9, die mono- oder biaxial im Verhältnis 1 : 1,5 bis 1 : 3,0 gereckt sind.

17. Verbundfolien aus eine Folie gemäß Anspruch 14 und einer Folie aus einem anderen Kunststoff.

18. Verwendung der Folien gemäß Anspruch 14 als Membran für die Gaspermeation.

## Claims

1. Dihydroxydiphenyl cycloalkanes corresponding to the formula

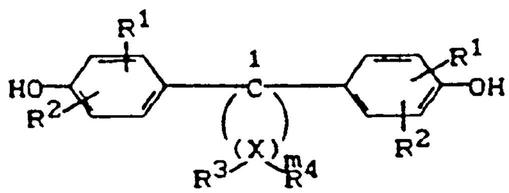 (I),

in which
$R^1$ and $R^2$ independently of one another represent hydrogen, halogen, $C_1$-$C_8$ alkyl, $C_5$-$C_6$ cycloalkyl, $C_6$-$C_{10}$ aryl and $C_7$-$C_{12}$ aralkyl,
m = 4 or 5,
$R^3$ and $R^4$ may be individually selected for each X and independently of one another represent hydrogen or $C_1$-$C_6$ alkyl and

X represents carbon, with the proviso that, at at least one atom X, both $R^3$ and $R^4$ are alkyl.

2. Dihydroxydiphenyl cycloalkanes corresponding to formula (I) as claimed in claim 1, characterized in that, in formula (I), neither of the two X atoms in the α-position to C-1 is dialkyl-substituted.

3. Dihydroxydiphenyl cycloalkanes corresponding to formula (I) as claimed in claim 2, characterized in that, in formula (I), a dialkyl-substituted X-atom is in the β-position to C-1.

4. 1,1-Bis-(4-hydroxyphenyl)-3,3,5-trimethyl cyclohexane.

5. A process for the production of the dihydroxydiphenyl cycloalkanes corresponding to formula (I)

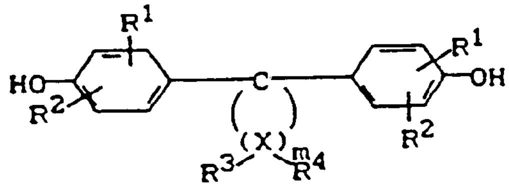

(I),

in which X, $R^1$, $R^2$, $R^3$, $R^4$ and m are as defined in claim 1, characterized in that phenols corresponding to formula (V)

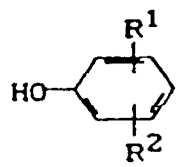

(V)

in which

$R^1$ and $R^2$ are as defined for formula (I), are reacted with ketones corresponding to formula (VI)

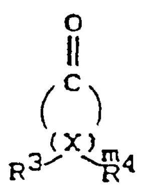

(VI)

in which

X, m, $R^3$ and $R^4$ are as defined for formula (I), in a molar ratio of (V) : (VI) of from 2 : 1 to 10 : 1 and preferably in a molar ratio of from 2.5 : 1 to 6 : 1 at temperatures in the range from – 30°C to 300°C and preferably at temperatures in the range from – 15 to 150°C and under pressures of 1 to 20 bar and preferably 1 to 10 bar and in the presence of acidic catalysts and optionally in the presence of co-catalysts and/or solvents and/or dehydrating agents.

6. The use of the diphenols corresponding to formula (I) claimed in claim 1 for the production of high molecular weight, thermoplastic aromatic polycarbonates.

7. A process for the production of high molecular weight aromatic polycarbonates from diphenols, optionally chain terminators and optionally branching agents by the known methods for the production of polycarbonates, preferably by the two-phase interfacial process, characterized in that the diphenols of formula (I) in claim 1 are used as the diphenols in quantities of 100 mol-% to 2 mol-%, based on the total mols of diphenols used.

8. High molecular weight, thermoplastic aromatic polycarbonates having weight average molecular weights $\overline{Mw}$ of at least 10,000 obtainable by the process claimed in claim 8.

9. High molecular weight, thermoplastic, aromatic polycarbonates having $\overline{Mw}$ values (weight average molecular weights) of at least 10,000 which contain bifunctional carbonate structural units corresponding to formula (Ia)

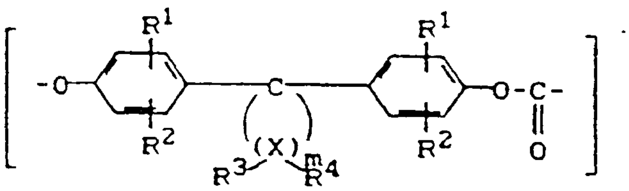

(Ia)

in which
X, $R^1$, $R^2$, $R^3$, $R^4$ and m are as defined for formula (I) in claim 1, in quantities of 100 mol-% to 2 mol-%, based on the total quantity of 100 mol-% of difunctional carbonate structural units in the polycarbonate.

10. Polycarbonates as claimed in claim 9, characterized in that they contain the carbonate structural units corresponding to formula (Ia) in quantities of 100 mol-% to 5 mol-%.

11. Polycarbonates as claimed in claim 9, characterized in that they contain quantities complementary to 100 mol-% of difunctional carbonate structural units corresponding to the formula

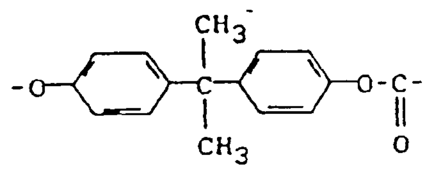

12. Polycarbonates as claimed in claim 9 containing structural units of formula (Ia) correspond to formula (Ic)

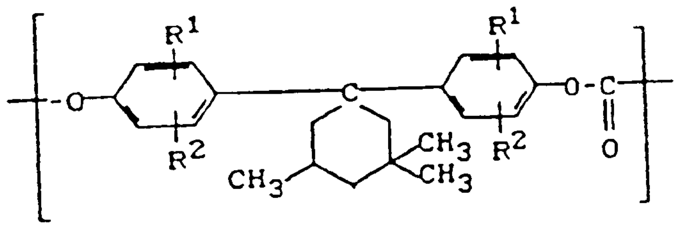

(Ic)

in which
$R^1$ and $R^2$ are as defined for formula (Ia) in claim 9.

13. Polycarbonates as claimed in claim 9 containing structural units corresponding to formula (IIa)

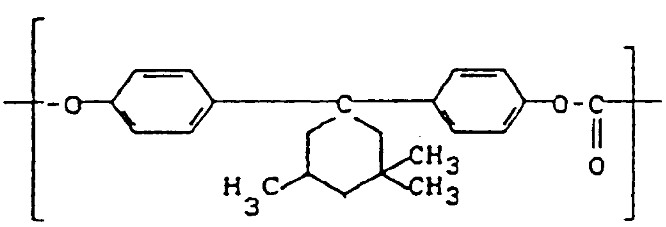

(IIa)

14. Films of the polycarbonates claimed in claim 9.

15. 1 to 1500 μm thick films of the polycarbonates claimed in claim 9.

16. 1 to 1500 μm thick films of the polycarbonates claimed in claim 9 monoaxially or biaxially stretched in a ratio of 1 : 1.5 to 1 : 3.0.

17. Composite films of a film as claimed in claim 14 and a film of another plastic.

18. The use of the films claimed in claim 14 as gas permeation membranes.

**Revendications**

1. Dihydroxydiphénylcycloalcanes de formule

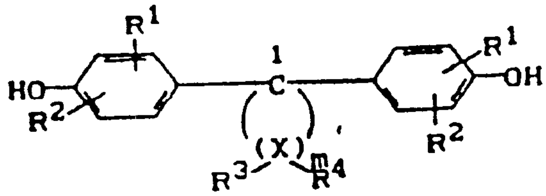 (I),

dans laquelle

$R^1$ et $R^2$ désignent indépendamment l'un de l'autre de l'hydrogène, un halogène, un radical alkyle $C_1$-$C_8$, cycloalkyle $C_5$-$C_6$, aryle $C_6$-$C_{10}$ et aralkyle $C_7$-$C_{12}$,

m désigne 4 ou 5,

$R^3$ et $R^4$, pouvant être choisis individuellement pour chaque X et indépendamment l'un de l'autre, désignent de l'hydrogène, un radical alkyle C1-C6 et

X désigne le carbone à condition que, sur au moins un atome X, $R^3$ et $R^4$ désignent simultanément un radical alkyle.

2. Dihydroxydiphénylcycloalcanes de formule (I) selon la revendication 1 caractérisés en ce que, dans la formule (I), les deux atomes X en position alpha par rapport à C-1 ne sont pas dialkylsubstitués.

3. Dihydroxydiphénylcycloalcanes de formule (I) selon la revendication 2 caractérisés en ce que, dans la formule (I), un atome X dialkylsubstitué est en position bêta par rapport à C-1.

4. 1,1-bis-(4-hydroxyphényl)-3,3,5-triméthylcyclohexane.

5. Procédé de préparation de dihydroxydiphénylcycloalcanes de formule (I)

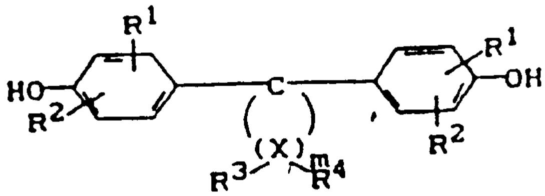 (I)

dans laquelle

X, $R^1$, $R^2$, $R^3$, $R^4$ et m ont la signification donnée dans la revendication 1, caractérisé en ce que des phénols de formule (V)

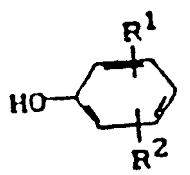 (V)

dans laquelle

$R^1$ et $R^2$ ont les significations spécifiées pour la formule (I) sont amenés à réagir avec des cétones de formule (VI)

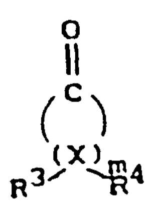 (VI)

dans laquelle
X, m, $R^3$ et $R^4$ ont les significations spécifiées pour la formule (I), dans un rapport molaire (V) : (VI) de l'ordre de 2 : 1 à 10 : 1, de préférence de 2,5 : 1 à 6 : 1 à des températures de – 30°C à 300°C, de préférence de – 15°C à 150°C et des pressions de 1 à 20 bar, de préférence de 1 à 10 bar en présence de catalyseurs acides et éventuellement de cocatalyseurs et/ou de solvants et/ou d'agents déshydratants.

6. Mise en oeuvre des diphénols de formule (I) de la revendication 1 pour la préparation de polycarbonates aromatiques thermoplastiques de poids moléculaire élevé.

7. Procédé de préparation de polycarbonates aromatiques thermoplastiques de poids moléculaire élevé à partir de diphénols, éventuellement d'agents de rupture de chaîne et éventuellement d'agents de ramification selon les méthodes connues de préparation de polycarbonates, de préférence selon le procédé à l'interphase caractérisé en ce que les diphénols utilisés sont ceux de la formule (I) de la revendication 1 dans des quantités de 100% à 2% en moles par rapport à la quantité molaire totale de diphénols utilisés.

8. Polycarbonates aromatiques thermoplastiques de poids moléculaire élevé avec un $M_{\omega}$ (poids moléculaire en moyenne pondérale) d'au moins 10.000 obtenus selon le procédé de la revendication 8.

9. Polycarbonates aromatiques thermoplastiques de poids moléculaire élevé avec un $M_{\omega}$ (poids moléculaire en moyenne pondérale) d'au moins 10.000 qui contiennent des unités de structure de carbonate à deux fonctions de formule (Ia)

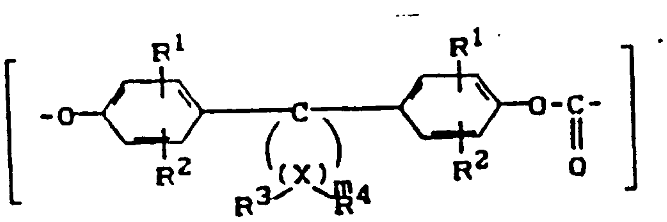
(Ia)

dans laquelle
X, $R^1$, $R^2$, $R^3$, $R^4$ et m ont les significations spécifiées pour la formule (I) de la revendication 1 dans des quantités de 100% à 2% en moles par rapport à la quantité totale de 100% en moles d'unités de structure de carbonate à deux fonctions dans le polycarbonate.

10. Polycarbonates selon la revendication 9 qui contiennent les unités de structure de formule (Ia) dans des quantités de l'ordre de 100% à 5% en moles.

11. Polycarbonates selon la revendication 9 qui contiennent les quantités complémentaires pour parvenir à 100% en moles d'unités de structure à deux fonctions de formule

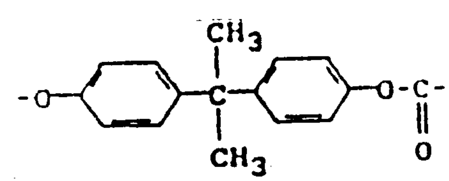

12. Polycarbonates selon la revendication 9 contenant des unités de structure de formule (Ic)

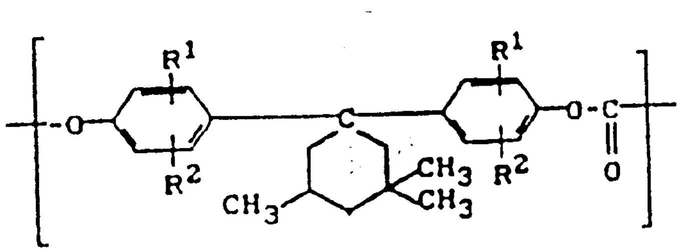
(Ic)

dans laquelle
$R^1$ et $R^2$ ont les significations spécifiées pour la formule (Ia) de la revendication 9.

13. Polycarbonates selon la revendication 9 contenant des unités de structure de formule (IIa)

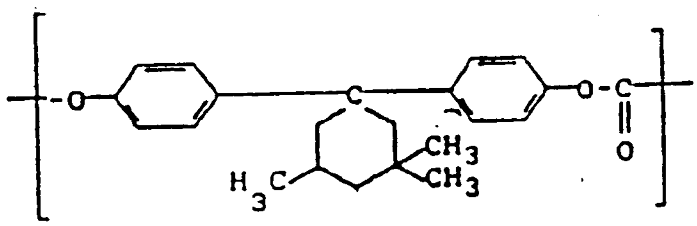

(IIa)

14. Feuilles en polycarbonates selon la revendication 9.

15. Feuilles d'une épaisseur de 1 à 1.500 µm en polycarbonates selon la revendication 9.

16. Feuilles d'une épaisseur de 1 à 1.500 µm en polycarbonates selon la revendication 9 qui sont étirées monoaxialement ou biaxialement dans un rapport de 1 : 1,5 à 1 : 3,0.

17. Feuilles stratifiées à partir d'une feuille selon la revendication 14 et d'une feuille en une autre matière plastique.

18. Utilisation des feuilles selon la revendication 14 comme membrane pour la perméation aux gaz.